# EUROPEAN PATENT APPLICATION

(11) **EP 4 098 242 A1**
(43) Date of publication of application: **07.12.2022**
(21) Application number: 20916766.7
(22) Date of filing: 29.01.2020
(51) Int. Cl.: A61K 8/19, A61K 8/24, A61K 8/33, A61K 8/55, A61K 8/64, A61K 8/96, A61Q 11/00

(54) **ORAL COMPOSITION WITH SYNERGISTIC ASSOCIATION OF ORGANIC AND INORGANIC COMPONENTS FOR COMPLETE MAINTENANCE OF ORAL HEALTH, METHOD FOR OBTAINING SAME AND USES**

(71) Applicant: Teixeira, Marcelo Rodrigues, 86055-736 Londrina - PR (BR); Rabbit Indústria e Comércio de Produtos de Higiene Pessoal Ltda, 86046-140 Londrina - PR (BR)
(72) Inventor: VILHENA, Fabiano Vieira, 12243-001 São José dos Campos/SP (BR)
(74) Representative: Hernandez Lehmann, Aurelio
(86) International application number: PCT/BR2020/050018
(87) International publication number: WO 2021/151174

(57) **Abstract**

This invention describes an oral composition, the variations, uses and production method thereof, wherein it is an acidified bioactive complex obtained from association of salts, organic compounds, silicon compounds and phosphates which, when in the mouth, is electrochemically attracted by the tooth, bonding to it and causing ionization of calcium from the dental structures, due to its acidic characteristics. Once bonded to the tooth, the complex gathers dispersed particles in the buccal medium, condensing them and, mainly from calcium, forming a hybrid layer containing silicon-enriched hydroxyapatite. This hybrid layer remineralizes the tooth enamel surface, functioning as a protective shield over the tooth, protecting against the everyday acidic challenges, when the dentin is exposed, this layer obliterates the dentin tubules, relieving the pain caused by dental sensitivity. The layer formation occurs in a self-etching manner, in other words, each application of the oral composition forms a new three-dimensional layer on top of the previous one; Thus, the formation of minerals in acidic medium, exactly in the same medium where mineral loss occurs, enables the formation of the hybrid layer, which enables the product to provide full maintenance of oral health.

## Description

This patent invention application refers to an unprecedented "ORAL COMPOSITION WITH SYNERGISTIC ASSOCIATION OF ORGANIC AND INORGANIC COMPONENTS FOR FULL MAINTENANCE OF ORAL HEALTH, PROCESS FOR OBTAINING AND USES THEREOF", which describes a composition and variations thereof with synergistic association of inorganic and organic components obtained through interaction between 0.1% and 7% salts, acids, silicon-based compounds and other carbon-containing compounds, in a medium with minor water concentration and acidic pH between 2 and 5.5. Said composition, when in contact with the buccal medium, has shown strong affinity with dental tissue, capable of acidifying the buccal medium during use and favoring the maintenance of an acidic to slightly alkali pH (5.00 to 7.5 pH in the mouth, and likely higher in the tooth/reaction interface), which accelerates the bonding, formation, and deposition of minerals, more precisely, *in situ* formation of a hybrid (organic and inorganic) microporous biomaterial layer on the dental structure, with pore diameters likely lower than 2 nanometers. The layer formed is insoluble in water at mouth temperature and its formation occurs regardless of fluoride ions, with its deposition process on the dental structure (enamel, dentin, cementum) started by the bonding of a silicon-based complex produced and stored in single-phase and/or multi-phase containers, with components that comprise the aforementioned invention being microlyzed or not (nanometric scale). The layer of hybrid biomaterial formed through use of the composition promotes full maintenance of oral health. Regarding restorative dentistry, it provides better adhesion of dental adhesives, in addition to dental cementum used in teeth restoration. Regarding preventive and aesthetic dentistry, the hybrid layer formed has active effect in functional and aesthetic repairing and protection; these actions are known by remineralization, protection against cariogenic and microbial processes with late release of active ingredients; teeth whitening, both optical, due to the adhesion in the particle layer, and chemical-mechanical in removal of pigments, protection against erosive processes, protection against corrosion, acidic attacks and relief of dental sensitivity through obliteration of dentin tubules.

### FIELD OF APPLICATION

This invention belongs to the category of human needs, to the field of dentistry and hygiene and, more specifically, to dentistry preparations for describing an oral composition comprising synergistic associations of its components, providing increased adhesion of dental adhesives on teeth, active effect in protection and recovery, in addition to functional and aesthetic repairing, after use of the composition.

### REASONING

The behaviors of modern society, such as consumption of sugary and acidic foods, stress, teeth tension and use of orthodontic appliance, are directly related to the occurrence of problems in the mouth cavity, among which cavities, erosion and dental sensitivity are the most common. Formulations for oral care currently need to adapt to the reality of society behavior. Ongoing improvement of technologies is required in this field in face of the challenges: prevention, protection and repairing against cavities and dental erosion, in addition to other mouth problems, as well as pain relief or eradication and diminishing of dental sensitivity. In addition, aesthetics and functionality/durability of dentistry procedures are increasingly required from professionals by patients.

Despite several technologies already developed, recent studies have demonstrated the inefficiency of bioactive toothpastes in relation to the challenges of modern life. According to João-Souza and collaborators (2017), toothpastes are not enough to solve the issues of dental erosion and hypersensitivity, and still within this issue, Lonta and collaborators (2019) state that no toothpastes supposedly with anti-erosion properties actually performs such effect. Regarding the destructive processes of dental tissue (demineralization) among which are dental cavities and dental erosion, fluorine (F) became the main active element incorporated in products used in counteracting these problems.

However, according to some studies, products once widely used for prevention of the aforementioned problems, such as fluorine, are of limited effectiveness. According to Schmidlin and collaborators (2016), in face of the fluorine limitation, the action of bioactive agents, such as those added to toothpastes, are frequently required in order to promote remineralization. Another current problem that should be highlighted is that child populations have been experiencing a large increase in Molar Incisor Hypomineralization - MIH, in which the malformation of enamel facilitates the development of the cariogenic process, in addition to exposure of the dentin, adjacent tissue to the enamel. Such exposure leads to extremely painful processes through the communication via dentin tubules of the buccal medium directly with the dental pulp. Regarding this issue, it is known that toothpastes that claim to have desensitizing properties just barely alleviate, if at all, the pain in children, therefore more expensive and professionally performed procedures are required in a doctor's office, with application of sealing agents and professional use desensitizing agents, and in more severe cases, tooth extraction and restoration. In face of the foregoing, this invention provides a formulation that fully addresses the maintenance of oral health. Regarding functionalities and components, it is possible to say, based on an extensive research history, that there is nothing similar available. According to the most recent review of the subject (Leal & Takeshita et al., 2019 - Pediatric Restorative Dentistry), mechanisms for which fluorine acts in demineralization and remineralization processes of enamel and dentin are already well established and known for relying on calcium and phosphate ions available when in the presence of fluorine. Phosphate and calcium-based agents have been investigated in isolation or in association with fluorine, in order to enhance the remineralizing effect, which would lead to increased disease protection and prevention, mainly acting in the replacement of pure hydroxyapatite for a less soluble one. Hydroxyapatite (Ca₁₀(PO₄)₆(OH)₂) is a mineral generated in the tooth that may undergo transformations and become more insoluble in face of everyday challenges withstood by the tooth. Table 1 shows possible modifications of hydroxyapatite, according to Da Silva and collaborators (2012);

**Table 1: Examples of substitutions in apatite.**

| Substitutions | Theoretical chemical formulations |
|---|---|
| | |

| Site Ca⁺² | |
|---|---|
| Mg⁺² | Ca₁₀₋ₓMgₓ(PO₄)₆(OH)₂) |
| Zn⁺² | Ca₁₀₋ₓZnₓ(PO₄)₆(OH)₂) |
| Sr⁺² | Ca₁₀₋ₓSrₓ(PO₄)₆(OH)₂) |
| Ag⁺² | Ca₁₀₋ₓAgₓ(PO₄)₆(OH)₂) |
| | |

| Site B (PO₄⁻³) | |
|---|---|
| CO₃⁻² | Ca₁₀₋ₓ(PO₄)₆₋ₓ(CO₃)ₓ(OH)₂₋ₓ |
| SiO₄⁻⁴ | Ca₁₀₋ₓ(PO₄)₆₋ₓ(SiO₄)ₓ(OH)₂₋ₓ |
| HPO₄⁻² | Ca₁₀₋ₓ(PO₄)₆₋ₓ(HPO₄)ₓ(OH)₂₋ₓ |
| | |

| Site A (OH⁻) | |
|---|---|
| F⁻ | Ca₁₀₋ₓ(PO₄)₆(OH)₂₋₂ₓF_{2X} |
| Cl⁻ | Ca₁₀₋ₓ(PO₄)₆(OH)₂₋₂ₓCl_{2X} |
| CO₃⁻² | Ca₁₀₋ₓ(PO₄)₆(OH)₂₋₂ₓ(CO₃)₂ₓ |

The main modification in hydroxyapatite, widely advocated in dentistry, is the addition of F on site A, transforming it in a less soluble mineral known as fluorapatite and/or fluorinated apatite. In general, hydroxyapatite is a calcium phosphate compound. Phosphate compounds help in tooth remineralization, rendering it less soluble. Some of these compounds are used as calcium and phosphate sources, in order to increase saturation of the buccal medium in relation to hydroxyapatite, which includes: nanohydroxyapatite, bioactive glass containing sodium and calcium phosphosilicate (Novamin^{®}), double chamber system in which calcium salts are separated by a plastic compartment for sodium fluoride and phosphate salts (Enamelon), dicalcium phosphate dihydrate (DCPD), casein phosphopeptides (CPP) bonded to amorphous calcium phosphate (ACP), functionalized tri-calcium phosphate and glycerol phosphate calcium. This way, an ideal formulation should combine the ability of improving remineralization and decreasing mineral loss, erosive wear, and dentin sensitivity (Leal & Takeshita et al., 2019: Pediatric Restorative Dentistry). Biomaterials were always used for substitution, repair, and regeneration of hard dental tissue. As research progressed, significant development in the field of dental materials was observed, and either new materials were provided, or improvement of already existing materials. As opposed to the development of bioinert materials, recent research on hard tissues have reached the development and further applications of bioactive materials, with the development of bioactive glass as its main characteristic. Originally discovered in 1969, bioactive glasses have provided a reliable alternative to inert implant materials due to its ability to form a stable bond with host tissues, subsequently inducing remineralization, especially for hard dental tissue (Khalid 2017 - Bioactive Glasses and their Applications in Dentistry). Several works in the literature have been applying the use of silicon-based materials as a preceding material or even as direct surface coating materials. As such, silicates, bioglasses and other silicon sources are widely researched and used. According to Da silva and collaborators (2012), data from the literature show that incorporating Si in the HA-Hydroxyapatite structure increases material bioactivity. SiHA-based ceramics are able to develop a new biological apatite phase layer on the surface in contact with physiological fluids more rapidly than pure HA. Studies involving cell culture describe that the proliferation of bone cells and their differentiation are more important over the SiHA surface than HA. One of the materials constituted of silicon is silica. Silica is an inorganic polymer, consisting of siloxane groups (Si-O-Si) inside, and silanol groups (Si-OH) on its surface. Silica forms one of the most used inorganic substance classes as support of a variety of systems with different applications. Its main studied property is related to its surface, which raises interest regarding the study of its molecule or ion adsorption properties. For Benvenutti and collaborators (2009), new innovative materials and the improvement of already existing materials is part of the everyday challenge of science evolution. Combined organic and inorganic components result in new materials known as organic-inorganic hybrids. Said materials associate their inherent physiochemical properties and combine them, synergistically forming highly promising materials and harnessing the best characteristics of each. Hybrids with silica as their inorganic component, also called silica-based hybrids, are the most important, studied and technologically applied, featuring several applications such as: catalysts, drug carriers, protective coating, among others.

Among silica particles, amorphous silica stands out due to its extraordinary advantages, including easy surface modification, low cost, and low toxicity. Therefore, it has been used in several applications, including cosmetics, food, and medical diagnosis. The polymerization and precipitation of silica occur in a wide range of environmental and industrial processes: ceramic and catalytic applications, dimensioning of water heaters, biomineralization, application of coatings to improve adhesion and moisturizing properties, etc., making silica polymerization an intensely studied subject. Due to the nature of applications, the research of silica polymerization and precipitation refer to polymerization in slightly acidic to basic solutions, in which silica polymerization begins with condensation in cyclic oligomers, which grow into three-dimensional polymeric particles (Gorrepati, 2010). In Gorrepati's work (2010), silica polymerization at pH 7 (neutral) was demonstrated, and a relation between salts at the silica polymerization speed was found. The author mentions that polymerization exponentially increases with the increase in acid concentration, as well as addition of salts, accelerating the polymerization. These findings are in line with Crear and collaborators (1981) which have confirmed the speed increase of silicon polymers with addition of salts. In dentistry, use of fluorine is widely advocated due to its performance regarding the formation of calcium fluoride and fluorapatite, in the prevention of cavities.

However, another important and widely applied role of the fluoride anion is as a reaction catalyst, which may be used both in acidic and basic media and has shown to be a highly efficient catalyst for silica-based hybrid gelling process. Although the action mechanism is not fully understood, there is consensus that fluoride, as it is a very small anion and easily diffused in the system, starts the process through a nucleophilic attack on silicon, coordinating with it and providing subsequent reactions. Fluoride is commonly used in the form of HF, as it was observed that, in saline form, such as NaF, the metallic cation electrostatically interacts with the alkoxide groups, inhibiting the process of polycondensation (Bevenutti et al., 2009). This invention advocates precisely the opposite, with sodium fluoride (NaF) as to avoid polycondensation, since it is known that the absence of polycondensation turns the hybrid layer less organic, microporous, and more resistant to acidic attack. Such action is evident in the work of Pavan and collaborators (2003), such as the presence of Na+ (when NaF is used) has led to a decrease in final organic content of materials. This effect was interpreted as an inhibition of the polycondensation of the organosilane, possibly due to the interaction of Na+ with the SiO⁻ groups of hydrolyzed organosoils, which has also changed the morphology, rendering it less porous. Silica solubility is constant in the 2-9 pH range, where the ideal pH for silica precipitation as a colloid is near 4.5. (Gomes, 2018). It is known that colloidal and attrition behaviors of silica perform an important role and are much more complex than expected for materials with smooth and chemically inert surfaces (Donose *et al*., 2005). Also, according to Donose and collaborators (2005), the adsorption of hydrated cations may also carry out an important role in adhesive interaction between surfaces. Various authors have studied the adhesion of silica to dental structures and shown strong bonding with the tooth (Addy *et al*. 1989, Perdigão *et al*. 1994, Lee *et al*. 2007).

In a classic study, Lee and collaborators (2007) have shown the eradicating capacity of dentin tubules via a dentifrice formulation containing n-CAPs (microlyzed carbonated apatite) and silica. In another classical study, Perdigão and collaborators (1994) have shown that the acid used to prepare the dental structure for adhesion of restorations, acid thickened with silica, has bonded strongly between silica and the tooth, being impossible to remove after washing. It may also be said that silica glued both to the enamel and the dentin has not affected the bonding strength. On the opposite, a slight increase in bonding strength was experienced, showing a strong connection between silica and the dental structures. In the works of Giordano and collaborators (2016), it was demonstrated that the classic sol-gel deposition, hydrolysis, or condensation of silicon alkoxides takes place under acidic or alkaline conditions, respectively. As all species are hydrolyzed in the initial reaction stage, they may condensate to form minor oligomer species (groups) with reactive Si-OH groups. Under these conditions, reactions in terminal silicon atoms are favored. This leads to polymeric gels; in other words, small clusters undergo condensation reactions between each other to yield a network similar to polymers with small pores. In their experiment, authors made use of EADS - Electrochemically Assisted Deposition of Sol-Gel, equipment based in local electrochemical generation of OH⁻ or H₃O⁺ to increase the rates of sol-gel reactions; this means other parameters must be considered, including cell geometry, work potential, ion conductivity, etc. In particular, the choice of the initial solution is a critical point for the electrodeposition method, as it is known that the reagents perform different roles and functions during deposition. When EADS is activated through localized reduction of the reasonably acidic electrolyte, polycondensation takes place on the surface of the electrolyte-electrode due to the local increase in OH concentration (alkali catalysis). OH⁻ ions are generated from the electrochemical reduction of H₃O⁺ and/or H₂O, however, H₃O⁺ and H₂O not only take part in the reduction reaction on the surface of the working electrode but are key variables in the sol-gel process; in fact, water accelerates polycondensation of the silica gel and H₃O⁺ allows for a quicker hydrolysis of the alkoxide groups. Therefore, the H₃O⁺ concentration must be kept as low as possible and, as a consequence, an electrolyte is required to dampen the ohmic decrease in the solution. In this context, the small amount of water included in the invention stabilizes the complexes, preparing it so that when in contact with the saliva, calcium and proteins, precipitation and formation of the hybrid layer is possible. Fowler and collaborators (2005) have demonstrated in their work the surfactants' capacity of affecting crystallization and nucleation processes, as well as mineral growth of calcium phosphates. Among the studied surfactants is sodium laureth sulfate. As a conclusion, they found a significant influence of organic assembly in formation calcium phosphate materials. Reports of polyphosphates with film forming characteristics are also present in the literature. In the work of Morsh and collaborators (2018), polyphosphates, among which are pyrophosphates, work by forming a surface protection film on tubes to inhibit corrosion. The functioning of the hybrid layer is also enabled by the use of polyethylene glycol (PEG), which has a variety of relevant properties, among which: insolubility in water at high temperatures, formation of complexes with metallic cations, high mobility with great volume power excluded in water, precipitating agent for proteins and nucleic acids. PEG is subjected to ready-made chemical modifications and bonding to other molecules and surfaces, when connected to other molecules, PEG modulates the solubility and increases the size of bonded molecules. Benvenutti (2009) has observed that PEG macromers were easily functionalized in the preparation of hybrid silica-based materials, the process, albeit similar, involves the addition of new components that increase system complexity, in other words, molecular precursors of the organic component are also added. This invention, in addition to its other functions, is also a whitening agent, in the composition the dye is incorporated to the matrix containing silicon, therefore, in a molecular level distribution, so that the dye is trapped in closed pores of the silica matrix, keeping its optical properties, and also increasing the emission intensity (Benvenutti et al., 2009), thus functioning as optical whitening agents, enabling the emission of white light in the color spectrum.

Thus, it is observed that the pleaded composition fully addresses the maintenance of oral health, solving in an innovative way This issue in the current state of the art, regarding the proposition of oral compositions capable of promoting full maintenance of oral health. In face of the foregoing, it is possible to notice that This invention arises out of extensive research for development of an effective and innovative product, as well as its process for obtaining, in order to synergistically gather the properties of its components into a composition with remineralizing action, protection against cariogenic, acidic, microbial, and erosive processes, whitening properties and relief of dental sensitivity.

### PRIOR ART TO THE INVENTION

The current state of the art includes prior arts that describe dentifrice compositions aiming at improving dental sensitivity and remineralization. However, no art was found that provides full maintenance of oral health through a synergistic association of organic and inorganic components that generates an anti-demineralizing/regenerative action, whilst simultaneously providing repairing of the already demineralized area, also being a desensitizing and whitening composition. The trajectory of acidic dentifrice studies is extensive, studies carried out over 40 years ago have demonstrated the action of acidic dentifrices always in combination with fluorides, which occurs because fluorides become more reactive when acidified, with improved performance. In this sense, inventions that describe acidic compositions always feature their main active agent followed by fluorine. As for pH control of standard compositions, it is perceived that this is provided by phosphoric acid, which has shown to be the most suitable, since phosphate is key for the demineralization and remineralization processes, in addition to acidic pH (Leal & Takeshita et al., 2018).

Table 2 below shows some prior arts included in the current state of the art, further ahead in this document, other prior arts are also commented and compared to the composition pleaded in this invention.

**Table 2: Toothpaste related studies in the last 50 years. Extracted and translated from the book Pediatric Restorative Dentistry - Leal & Takeshita et al. 2018.**

| Authors (year of publication) | Study protocol | Main variables studied | Main results |
|---|---|---|---|
| Gerdin (1974) | *in vivo* | Cavity increase (dmfs) | 250 ppm F (pH 5.5) similar to 1000 ppm F related to cavity increase |
| Petersson *et al*. (1989) | *in vitro* | Absorption of fluorine in enamel | 250 ppm F (pH 5.5) similar to 1000 or 1500 ppm (neutral pH) |
| Negri and Cury (2002) | *in vitro* | Absorption of fluorine in enamel | 550 ppm F (pH 5,5) similar to positive control (1100 ppm F, neutral pH) related to fluorine with strong and weak bonds |
| Brighenti *et al*. (2006) | *in vitro* | Enamel demineralization | 550 ppm F (pH 5.5) similar to 1100 ppm F (neutral pH) |
| Alves *et al*. (2007) | *in vitro* | Enamel demineralization | 412 of 550 ppm F (pH 4.5) similar to 1100 ppm F (neutral pH) |
| Nobre dos Santos *et al*. (2007) | *in situ* | Enamel remineralization / Absorption of fluorine in enamel | 550 ppm F (pH 5.5) similar to 1100 ppm F related to remineralization of strongly bonded enamel and fluorine. |
| Olympio *et al*. (2007) | *in vivo* | Saliva concentration of fluorine | Dental cream (pH 5.5) containing 550 ppm of elevated concentrations of saliva fluorine in similar levels to positive control. |
| Buzalaf *et al*. (2009) | *in vitro* | Enamel wear (abrasiveness) | 550 ppm F (pH 4.5) similar to 1100 ppm F (neutral pH) related to fluorine concentrations in dental biofilm. pH reduction of the dentifrice has not affected concentration of nail use fluorine (that is, systemic effect) . |
| Vilhena *et al.* (2010) | *in vivo* | Cavity progression (dmfs) | 550 ppm F (pH 4.5) similar to 1100 ppm F (neutral pH) |
| Brighenti *et al.* (2013) | *in vitro* | Enamel remineralization | 550 ppm F (pH 4.5) similar (surface hardness) or higher (cross section hardness) than 1100 ppm F (neutral pH). |
| Moron *et al*. (2013) | *in vitro* | Enamel wear (erosion) | Lesser protective effect of 550 ppm F (pH 4.5) compared to 1100 ppm F (neutral pH). |
| Cardoso *et al*. (2014) | *in vivo* | Cavity progression and regression / Fluorine concentration in toenails | Cavity progression significantly lower and liquid increase to 550 ppm (pH 4.5) compared to 1100 ppm F neutral (Nyvad's criteria) . |
| | | | 550 ppm F (pH 4.5) with a significantly better performance than the 1100 ppm F neutral (QLF analysis) . |
| | | | Lower concentration of fluorine on toenail associated to toothpaste with low fluorine contents (in other words, systemic effect) . |
| Cardoso *et al*. (2015) | *in vitro in vivo* | Enamel demineralization / Absorption of fluoride in biofilm | Lesser effect of 550 ppm F (pH 4.5) in demineralization of enamel compared to 1100 ppm F neutral; 550 ppm F (pH 4.5) provided a significantly higher biofilm fluorine absorption compared to 1100 ppm F neutral. |
| Kondo *et al.* (2016) | *in vivo* | Fluorine levels in saliva and biofilm (solid and fluid phases) | Higher fluorine concentrations in the biofilm 1 hour after brushing with acidic dentifrices compared to its neutral counterparts, despite differences not being significant; |
| | | | The pH of toothpaste has not affected fluoride |
| Ortiz *et al.* (2016) | *in vitro* | Enamel demineralization and absorption of fluorine in enamel | 550 ppm F (pH 4,5) similar or superior effect to 1100 ppm F (neutral) in relation to fluorine with strong and weak bonds; lower effect against demineralization. |
| Veloso *et al.* (2017) | *in vivo* | Absorption of fluorine in biofilm | 750 ppm F (pH 4.5) similar to neutral 1100 ppm F 60 minutes after brushing |
| Campos *et al.* (2017) | *in vivo* | Fluorine concentration on toenails | 750 ppm F (pH 4.5) led to lower fluorine levels on toenails than 1100 ppm Neutral F |

Prior art PI0705195-6, titled "Liquid acidified dentifrice formulation with low fluorine concentration and its use", also developed by the inventor of this patent application, refers to a formulation of low fluorine concentration (550 ppm F) and acidic pH, where the amount of orthophosphoric acid used for adjusting pH is around 0.25%. Such concentration is justified by the fact that if the acid is added at a higher concentration, the dentifrice would feature a lower pH than optimal, namely 4.5. It should be noted that the amount of water in the product is around 25%. Prior art BR102013006807-1, titled "Ultrafunctional dentifrice and use of fluorinated agent association and protease inhibitor", also developed by the inventor of this patent application, describes a dentifrice formulation including fluoride in higher concentration than the previous one and protease inhibitors with lower pH, between 4.3 and 6; in order to increase product efficiency in controlling dental cavity, dental erosion, and periodontal disease. Also, in this case the amount of water is around 25%. application PCTBR2018050485, titled "Multifunctional diphosphate fluorinated dentifrice composition", also developed by the inventor of this patent application, describes a dentifrice formulation comprising a synergistic association of a small amount of water, a fluorinated agent and two phosphate compounds, granting anti-demineralization / regenerative action to the composition which, in contact with tooth minerals, prevents loss of calcium and phosphate, which takes place during the demineralization process, whilst promoting repairing of already demineralized areas in initial stages, in other words, at enamel level.

The composition also includes a desensitizing action, due to its CaF₂ precipitation ability, which obliterates dentin tubules. This invention is distinguished from the first two mainly due to the amount of fluorine, acid (pH even lower in This invention) and water, as well as the manner in which product actions take place. Regarding the third application, although both include higher acid concentrations, less water and similar amounts of fluorine, compositions are totally different. This invention provides a unique silicon-based complex, obtained through preparation of an acidic and/or high acid concentration solution in a non-aqueous environment (small water amount). The fluorine of This invention has an additional function, in other words, in addition to forming fluorinated compounds as in previous applications, it is a reaction catalyst for *in situ* formation (in the mouth) of a silicon-enriched mineral layer.

Thus, this invention is not "fluorine-dependent" such as the previous ones, and this catalyst element was replaced by other components in This invention. The main action mechanism of the prior arts mentioned is the formation of phosphated calcium fluorides, as well as fluorapatite (fluorine-enriched hydroxyapatite), in This invention the mechanism that takes place is the formation of a hybrid mineral layer (organic and inorganic), with silicon-enriched hydroxyapatite being the main component formed. The hybrid mineral layer (organic and inorganic), with silicon-enriched hydroxyapatite being the main component formed, is more acid-resistant than fluorine-enriched hydroxyapatite (fluorapatite), mainly when the mouth's pH drops below 4.5 where even fluorapatite is soluble. The current state of the art also features technologies for modifications in the dentin surfaces. Yang and collaborators (2014) would describe the surface modifications on the dentin to an "enamel-like", a structure similar to enamel, as a potential strategy for improving the durability of dentin adhesion of restorations and cementing, desensitizing and automatically repairing the dentin. The authors have portrayed the histological complexity of dentin, as well as the vulnerability of the layer formed between the tooth and the restoration/cementing adhesive system that counteracts the long-term effect of dentin bonding; at the same time, it is more likely that post-procedure sensitivity occurs after standard adhesive restoration. In this context, this invention features a composition, as well as a method for obtaining it, for forming a dentin similar to the enamel, simulating the enamel structure to attain satisfactory durability of dentin adhesion and good performance in prevention of post-procedure sensitivity. With the application of bidirectional mesoporous silicon bonding to the hydroxyapatite of the dentin itself and the nanorods of in vitro synthesized hydroxyapatite, a new functional enamel-like surface layer may be formed through ion deposition and regulation. The enamel-like surface, described by Yang and collaborators (2014), was obtained using the MSNs (MCM-41) - nanoparticulated mesoporous silica, same amount of MSNs located, separately calcium oxide (CaO) and phosphoric acid (H₃PO₄) mixed with distilled water, forming a paste that is uniformly rubbed three times onto the deproteinated surface of the dentin. After coagulation around 10 minutes, the remaining adhesive on the surface was smoothly washed with water spray for 30 seconds, leaving a thin white coating on the surface of the deproteinated dentin. During the coating procedure, ions Ca²⁺ and HPO₄²⁺ are easily and quickly released due to the high rate of surface area / MSN volume, resulting in formation of CaHPO₄·₂H₂O precipitates that firmly obstruct the tubules, acting as a barrier to prevent the breaking of fluids from tubules on the bonding surface. At the same time, the CaP nucleation also contributes for adhesion between MSNs and the underlying deproteinated dentin when the pH value increases. Last, but not any less important, the MSNs strongly bond to the HAP of the deproteinated dentin through a chemical reaction for generation of silicon stabilized tricalcium phosphate (Si-TCP), leading to the formation of a silicon-rich layer. Chiang and collaborators (2010) have portrayed in one of their studies the precipitation of calcium phosphate with potential treatment of dentin hypersensitivity, due to occlusion of dentin tubules. The authors highlight a new mesoporous silica biomaterial (nano CaO @ mesoporous silica, NCMS) containing nano-sized calcium oxide particles mixed with 30% phosphoric acid may efficiently occlude the dentin tubules and significantly decrease the dentin permeability. The supersaturated NCMS paste, containing Ca²⁺ and HPO₄²⁺ ion, was brushed on the dentin surfaces and the ions have spread uniformly on the dentin tubules and formed a CaHPO₄·₂H₂O precipitation with 100 µm depth. The results of the permeability tests to the dentin have shown that the new mesoporous material was showing a significant reduction in dentin permeability (p < 0.05), in comparison with other materials previously developed by the same team, such as DP-Bioglass, and a Seal & Protect^{®} commercial desensitizer. It should be mentioned that, in this case, the mesoporous silica described herein has no function of precipitating and helping in obliterating the tubules, as reported by the authors. The mesoporous silica referred herein is merely a calcium carrier. Prior art WO1994020064A1, entitled "Calcium phosphate hydroxyapatite precursor and methods for making and using the same", describes acidic calcium phosphate compositions, particularly useful and unique as orthopedic and dental cements and remineralizers, a method for obtaining them and kits for proper use. The compositions comprise tetra calcium phosphate prepared from a mixture with a calcium-phosphor proportion mixture lower than 2, or prepared and maintained in substantially anhydrous conditions before use or, preferably, both.

Compositions are converted into hydroxyapatite, when hardening and are reabsorbed and replaced by bone when implemented in contact with living bone tissue, providing distinct advantages in terms of cement resistance, hardening time, and reliability, among other properties. Prior art US5079298A, named "Silicon-containing polymer having comb-shape structure", describes a silicon-containing polymer with a comb-shaped structure, comprising a unit of an organopolysiloxane as the main chain and a vinyl polymer as the side chain. The polymer has excellent migration properties and is useful as an agent for providing water repellent properties, adhesion, and surface activity. Prior art WO2013052181A2, called "Functionalized silicones with polyalkylene oxide side chains", describes silicone polymers with grafted pending polyalkylene oxide side chains and, optionally, functionally reactive terminal groups. These resources become suitable for use in chemical, marine, biomedical and industrial applications, particularly those involving surface modifications. According to Lippert and colleagues (2013), virtually all formulations that include sodium phosphates (pyrophosphates, for example) in their composition, have basic and non-acidic characteristics. These formulations typically have a high pH, in order to avoid the hydrolysis of these phosphates, a desired condition by the manufacturers of the formulations included in the state of the art. In This invention, precisely the hydrolysis effect is intended, different from the products described in the state of the art, considering the acidic pH is key for reactions to occur, so that phosphates and sodium are provided. The use of silica and its activation, turning it bioactive and enabling the formation of a polymer to be applied in the health industry, is widely known in the state of the art. However, activation of silica from the proposed process, in an acidic medium, using NaF and condensing salts as components and the obtained product, in other words, a composition with synergistic association of organic and inorganic components, which enables the *in-situ* formation of a microporous hybrid mineral layer with silicon-enriched hydroxyapatite, corroborating with the inventive step and the novelty of the proposed invention. Prior art WO2008068247A1, named "Oral care product", provides an oral care product comprising a mesoporous calcium silicate biomaterial (MCBS) dispersed in a polymeric material matrix. The document describes that, due to current lifestyles with increase in consumption of acidic food and drinks, dental erosion is becoming more prevalent and common, and is believed to be one of the largest threats to teeth of the 21st century. Acidic food and drinks, such as fruit and fruit juices, may render the enamel more fragile and generate wear due to acidic attack. Thus, without effective intervention or treatment, dental erosion may cause dental sensitivity with intense pain since dentin tubules would be exposed after the wear and tear of the enamel surface. Currently, the remineralization usually takes place in the form of the addition of fluoride ions according to the following reaction scheme: Ca5(PO₄)3OH→Ca5(PO₄)3F. This way, within the context of This invention, a MCSB is an insoluble composite material in silica-calcium oxide: CaO-SiO₂-. The material is characterized as in a mesoporous state, in other words, a material with pores varying between 0.4 and 50 micra in diameter. The material may be in an amorphous or crystalline state. The term "insoluble" must be understood as insoluble in water at mouth temperature. Typically, "insoluble" materials have solubility lower than 0.01 mol/L at 25°C. Prior art WO2012078136A1, named "Dentifrice compositions containing calcium silicate", describes an oral hygiene composition which includes an effective amount of calcium silicate particles, with average diameter lower than 5 micra, so that they may obstruct the dentin tubules of teeth. An oral hygiene method is described which includes the application of the composition in the oral cavity of a patient in order to reduce or inhibit tooth hypersensitivity and attain other benefits. Prior art US9717929B2, named "Dentifrice compositions containing calcium silicate and a basic amino acid", refers to dentifrice compositions containing calcium silicate and a basic amino acid in free or saline form which comprises an effective amount of calcium silicate particles with average diameter lower than approximately 5 micra, so that the dentin tubules of the teeth may be obstructed. An oral hygiene method is described which includes the application of the composition in the oral cavity of a patient in order to reduce or inhibit tooth hypersensitivity and attain other benefits. Prior art WO2018033427A1, named "Oral care composition", refers to an oral care composition for to the method for reducing sensitivity and/or remineralization and/or whitening of the teeth. The oral care composition disclosed herein comprises calcium silicate, 1% to 20% in weight of a potassium phosphate salt, a tubule blocking enabler selected from calcium dihydrogen phosphate, calcium sulfate hemihydrate or its mixtures and a physiologically acceptable vehicle, in which calcium silicate and the potassium phosphate salt is present in a 10:1 to 1:5 weight ratio.

Prior art WO2015167488A1, named "Oral care composition containing silica and zinc citrate", describes a formulation containing especially prepared silica, a bioadhesive, zinc and an anti-pelletizer agent - tetrasodium pyrophosphate. The use of silica, in particular, anticipates its use in the obliteration of dentin tubules, more precisely the use of silica particles with an average particle size no larger than the diameter of a dentin tubule for occlusion of dentin tubules and treatment of dentin hypersensitivity, which was also provided and disclosed in the previous patent application US2009/0092562, which is also used in This application. In addition, polyvinyl methyl ether/maleic anhydride ("PVM/MA") is a bioadhesive agent for fixing the assets, for example, the action of zinc as an antimicrobial agent and the anticipation of TSPP - Tetrasodium Pyrophosphate as an anti-pelletizer agent. Although the prior art provides the use of some components from This invention, it is evident that it does not use the acid to prepare the pH around 2 to 5.5 of the pleaded composition; in addition, the prior art does not describe the water reduction to a minimal amount, near zero, which enables the production of an extremely reactive dental gel, and the need for maintenance of a low pH during the entire use process is also not described, as described in This invention. It should also be mentioned that fluorine is not a *sine qua non* condition for functioning of this invention. Still on the reactive term, the acidic pH in contact with the buccal medium (water) transforms the pleaded composition in This application that requires ions, which, when connected to the dental structures, is capable of forming a hybrid layer of inorganic (calcium, sodium, phosphate, silicon, among others) and organic elements (proteins, pigments, among others). There is still another important difference regarding TSPP - tetrasodium pyrophosphate, used in the prior art as an anti-pelletizer agent, with an entirely different function in This invention, to be a sodium source for layer condensation, and a phosphate source for remineralization and formation of hybrid layer. Prior art WO2012010520A1, named "Hydroxyapatite-binding nano- and microparticles for cavity prophylaxis and reduction of dental hypersensitivity", refers to nano/microparticles functionalized with a binding oligopeptide to hydroxyapatite (marked nano-microparticles), which may be used to close dental cavities, fissures, and dentin tubules, in order to prevent formation of cavities and reduction of dental hypersensitivity. Previous strategies of the same inventors described above for cavity prophylaxis covering the teeth (sealing of pits and fissures) with a silica layer based only in the application of an enzyme (Silicatein), which bonds to the dental surface and, afterwards, catalyzes the formation of silica.

It is evident that This invention aims at the use of silica as a prophylaxis method for oral health conditions as described in the prior art, however, this invention is not a silica oligopeptide, but a silicon complex in its composition, activated by an acidified preparation method which directly bonds to the tooth and does not require prior reaction or preparation with peptides. Prior art WO2013052181A2, called "Functionalized silicones with polyalkylene oxide side chains", describes silicone polymers with grafted pending polyalkylene oxide side chains and, optionally, functionally reactive terminal groups.

These resources become suitable for use in chemical, marine, biomedical and industrial applications, particularly those involving surface modifications. Below are some prior arts that describe the use of bioglass in order to reach the effects pleaded by This invention, which uses a silicon source in acidic medium associated to NaF and condensing salts, less activation and formation of an *in-situ* hybrid layer; in addition, it should be emphasized that not all prior arts report a whitening/bleaching effect of compositions, as provided by This invention. Prior art WO2011161422A1, called "Bioactive glass composition", describes a bioactive glass composition comprising one or more glasses containing SiO2, P2O5 and a fluoride, with SiO2 contents lower than 40% in mole, P2O5 contents at least 4% in mole and fluoride contents higher than 1% in mole. The bioactive glass or vitroceramic may be used in several medical applications, including dental applications, such as toothpaste.

Prior art WO2005063185A1, named "Compositions and methods for preventing or reducing plaque and/or gingivitis using a bioactive glass containing dentifrice", describes method and compositions for preventing and/or reducing plaque, plaque accumulation and/or gingivitis. For such, bioactive glass compositions are provided that prevent or reduce plaque, formation of plaque and/or gingivitis through use of low levels of small bioactive glass particles in amounts around 0.25 to 10% weight in non-aqueous formulations. Resulting non-aqueous compositions are effective in dentifrice products, stable, and in compliance with ISO (International Organization for Standardization) standards. Furthermore, said bioactive glasses containing non-aqueous compositions feature unexpectedly high levels of antimicrobial activity against oral pathogens. Prior art WO2019115601A1, called "Novel composition", refers to non-aqueous dentifrice compositions comprising a source of calcium ions and a source of phosphate ions, such as a bioactive glass, a moistener such as glycerin, a hydroxyethyl cellulose polymer and pyrogenic silica. The calcium source and the phosphate source, together, are predecessors of the *in-situ* formation of a desensitizing/remineralizing agent in the oral cavity teeth. The compositions are useful in teeth remineralization and treatment of dentin hypersensitivity. Prior art WO2019034325, called "Oral care composition", describes an oral care composition comprising a bioactive glass, a tubule blocking enhancer selected from dihydrogenated calcium phosphate, calcium sulfate hemihydrate or a combination thereof, a source of phosphate and a physiologically acceptable carrier, wherein the bioactive glass and the tubule blocking enhancer are included in 1:3 to 30:1 weight ratio, and wherein the phosphate source is trisodium phosphate, monosodium dihydrogen phosphate, disodium hydrogen phosphate, ammonium phosphate, diammonium hydrogen phosphate, ammonium dihydrogen phosphate, tripotassium phosphate, monopotassium dihydrogen phosphate, dipotassium hydrogen phosphate, or a mixture thereof. Prior art WO2014154874A2, called "Chlorine-containing silicate glasses and glass ceramics", describes a silicate glass containing chlorine, which comprises SiO₂, at least 0.5% in mole of metal chloride and at least 10% in mole of MgO, SrO, BaO and CaO combined. Prior art WO2018118911A1, called "Whitening dentifrice compositions with zinc core shell silica particles", describes components used for dental whitening, a zinc-bonded blue silica dye. Thus, it refers to a whitening dentifrice composition free of peroxide whitening agents, includes a blue dyeing agent, a zinc core shell silica particle (Zn-CSS) and an acceptable oral carrier, including a non-aqueous solvent and water. The blue dyeing agent includes, at least, a blue pigment and a blue dye, and has a blue to violet-blue at a hue angle in the CIELAB system ranging between 200 to 320 degrees. The Zn-CSS particle includes a silica core and a silica core shell engraved with a metasilicate, which is a zinc ion silicate and, optionally, a monovalent metallic ion. Prior art EP0951441B1, called "Silicon-substituted apatites and process for the preparation thereof", describes a process for obtaining apatite or hydroxyapatite substituted by synthetic silicon that comprises 0.1% to 5% in weight of silicon. Differently from This invention, the prior art enriches apatite or hydroxyapatite outside the organism, whereas in This invention, when in contact with the buccal medium, the pleaded oral composition reunites particles dispersed in the buccal medium, mainly condensing from calcium, forming a hybrid layer containing silicon-enriched hydroxyapatite. Prior art JP2012514573A, called "Silicate-substituted hydroxyapatite", describes a hydroxyapatite substituted by inorganic silicate. Differently from This invention, the prior art refers to obtaining silicate-substituted hydroxyapatite, whereas in This invention, silicon enrichment occurs already in the buccal medium, when in contact with the buccal medium, reuniting particles dispersed in the buccal medium, mainly condensing from calcium and forming a hybrid layer containing silicon-enriched hydroxyapatite.

Although the state of the art includes prior arts that describe dentifrice compositions capable of providing pain relief, promoting teeth remineralization, none provides a formulation that fully addresses the maintenance of oral health.

Table 3 below briefly compares the invention described in This patent application with dentifrices present in the state of the art, summarizing its main differences:

**Table 3: Comparison between the invention and dentifrices from the state of the art.**

| | Invention | Dentifrices present in the state of the art | |
|---|---|---|---|
| | | Acidic pH dentifrices | Neutral/basic pH dentifrices |
| In tube (Formulation) | -- Water | ± Water | ± Water |
| | ++ Acidic | ± Acidic | No Acid |
| | + Phosphate | ± Phosphate | ± Phosphate |
| | +Na, K, Zn | ± Na, K, Zn | ± Na, K, Zn |
| | + Silica | ± Silica | ± Silica |
| | ± Fluorine | Fluorine | ± Fluorine |
| | Acidified bioactive complex containing silicon, among other components | Silica | Bioglass, silica and silicates |
| | pH from 2 to 5.5 | pH 4.5 | pH 7.0 or higher |
| | | | |
| In mouth | + Phosphate | Phosphate | ± Phosphate |
| | pH 5.5 to 6.5 | pH 5.5 to 6.5 | pH 7 or higher |
| | ++ FCa₂ Phosphate | ++ FCa₂ Phosphate | ± FCa₂ Phosphate |
| | ++Hybrid Layer: Si,P, Ca, protein (with or without fluorine) | - | ++Hybrid Layer: Si, P, Ca, protein (with or without fluorine) |
| | | | |
| On tooth | ++ Enamel permeability | +Enamel permeability | Enamel permeability |
| | ++ Mineral precipitation | Mineral precipitation | ± Mineral precipitation |
| | Hybrid Layer: Si, P, Ca, proteins (with or without fluorine) | - | Hybrid Layer: Si, P, Ca, proteins (with or without fluorine) |
| | Formation of microporous layer - acidic medium | - | Formation of mesoporous layer - alkaline medium |
| | + Silica | ± Silica | ±Silica/bioglass |
| | Active layer (molecule release and adhesion) | - | ± Adhesion layer and molecule release |
| | | | |
| Results | + Remineralization | Remineralization | ± Remineralization |
| | + Hybrid Layer (protection/adhesion) | | Hybrid Layer (protection) |
| | + Hybrid layer anti-dental erosion | - | Hybrid layer anti-dental erosion |
| | + Antimicrobial | + Antimicrobial | + Antimicrobial |
| | Whitening | - | Whitening |
| | + Desensitization | ± Desensitization | ± Desensitization |

### OBJECTIVE OF THE INVENTION

This invention aims at providing an oral composition with synergistic association of organic and inorganic components, with actions for remineralization, protection against cariogenic, acidic, microbial and erosion processes, whitening and relief of dental sensitivity, capable of providing full maintenance of oral health.

### SUMMARY OF THE INVENTION

This invention describes an oral composition, its variations, uses and modes for obtaining.

This way, the composition is characterized by the synergistic association of inorganic and organic components that takes place during the obtaining process, more precisely, between interaction of salts, acids, polymers, and silicon-based compositions, in a medium with minor water concentration - between 0.1 and 7%, therefore, considered non-aqueous, in acidified pH between 2 and 5.5. When in contact with the buccal medium, the pleaded composition features a strong affinity with dental tissue, also acidifying it during use and maintaining an acidified to slightly alkaline oral pH (5.0 to 7.5), which favors and accelerates the bonding, formation and deposition of minerals, more precisely it favors the *in-situ* formation of a hybrid (organic and inorganic) and microporous (pore diameter lower than 2 nanometers) biomaterial layer. The hybrid layer formed is insoluble in water at mouth temperature and its formation is independent of fluoride ions, with its deposition process started by the bonding of a silicon-based complex on the dental structure (enamel, dentin, cementum). The presence of the hybrid layer, generated from the interaction of the composition with the dental structure elements, enables full oral health maintenance. Regarding restorative dentistry, it provides better adhesion of dental adhesives, in addition to dental cementum used in teeth restoration. Regarding preventive and aesthetic dentistry, the hybrid layer formed has active effect in functional and aesthetic repairing and protection; these actions are known by remineralization, protection against cariogenic and microbial processes (through a physical barrier and late release of active ingredients), teeth whitening (optical - by adhesion on the particle layer; and chemical-mechanical by removal of pigments), protection against erosive processes and relief of dental sensitivity through obliteration of dentin tubules.

### ADVANTAGES OF THE INVENTION

This invention features as its main advantages:
✔ Providing a composition with actions for remineralization, protection against cariogenic and microbial processes (late release of active ingredients), teeth whitening (optical - by adhesion on the particle layer; and chemical-mechanical by removal of pigments), protection against erosive processes and relief of dental sensitivity through obliteration of dentin tubules;
✔ Providing a composition with activity in low pH; which occurs due to the lack of water and presence of metallic cations. In the presence of a watery medium, the polymerized and active silicon form is ready to react with cations (calcium), since repulsion is dampened, thus enabling their attraction on the tooth;
✔ Providing a composition that contains active colloidal silica in acidic medium, differently from products commonly distributed that include colloidal silica with basic pH;
✔ Providing a composition with independent activity from fluorine, which, when present, not only has the role of improving the effect of the active ingredients, but also as an accelerator, which is not essential, and fluorine may be replaced by other accelerators, such as sodium pyrophosphate, which also exercises the function of an anticorrosion agent.
✔ Providing a composition with an even more acidic pH (between 0.01 and 30% more acidic, compared to the original product), with no detrimental effects to its functions, through addition of acid in higher concentration, such as 37%, from a separated tube;
✔ Providing a process for obtaining an oral composition capable of correcting a common issue in the technical field, which is the hardening of the mixture inside the reactor and the product tube itself;
✔ Providing a product that is not fluorine-dependent, in order to enable the enamel remineralization process, considering that this action is enabled by the silicon included. Thus, the fluorine, when included, also ends up having the role of a polymerization reaction catalyst of the acidified bioactive complex, the oral composition described;
✔ Providing a product that, differently from the known techniques, especially Novamin^{®}, NR5, among others, do not necessarily require the calcium element in its composition, considering that the reaction with these elements takes place in the buccal medium, where they are naturally present. However, a calcium supplementation is able to improve the reaction, even though it is not a *sine qua non* condition to promote formation of a hybrid layer on the tooth.

### DESCRIPTION OF THE DRAWINGS

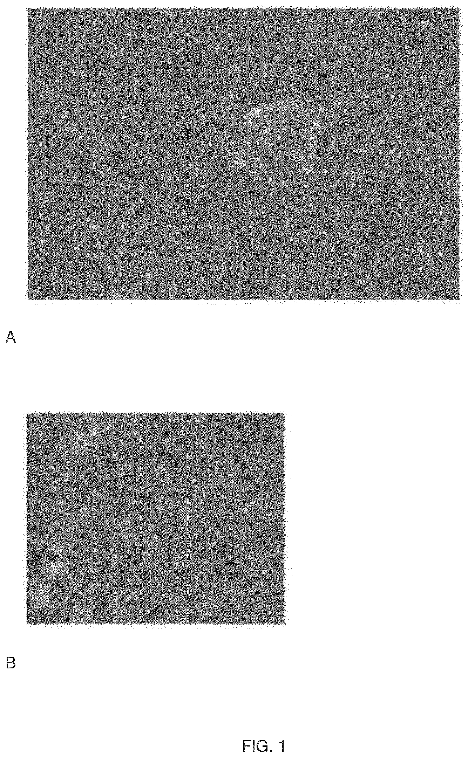

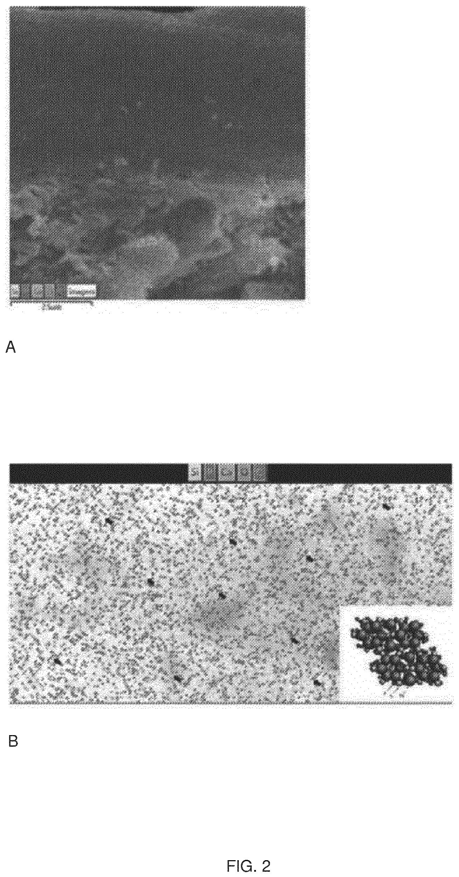

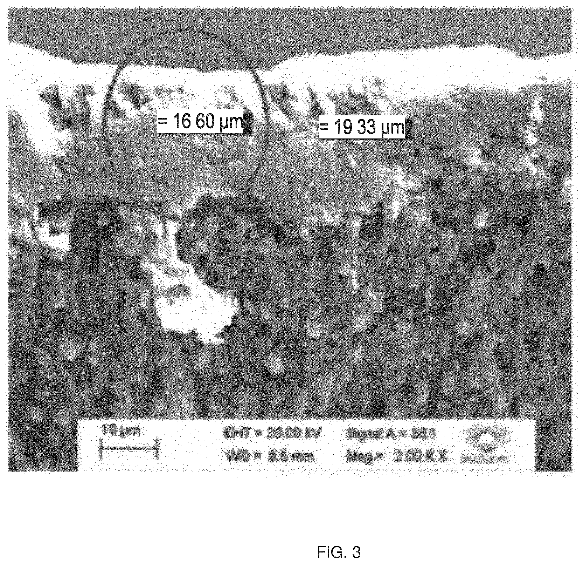

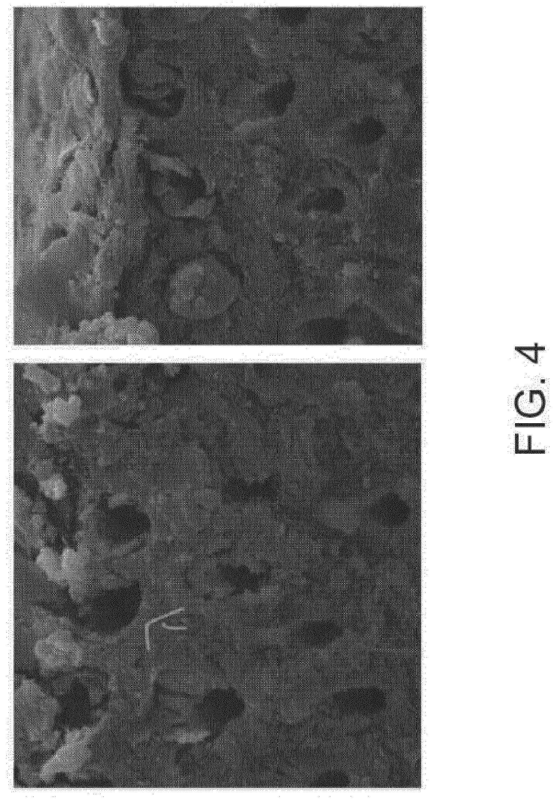

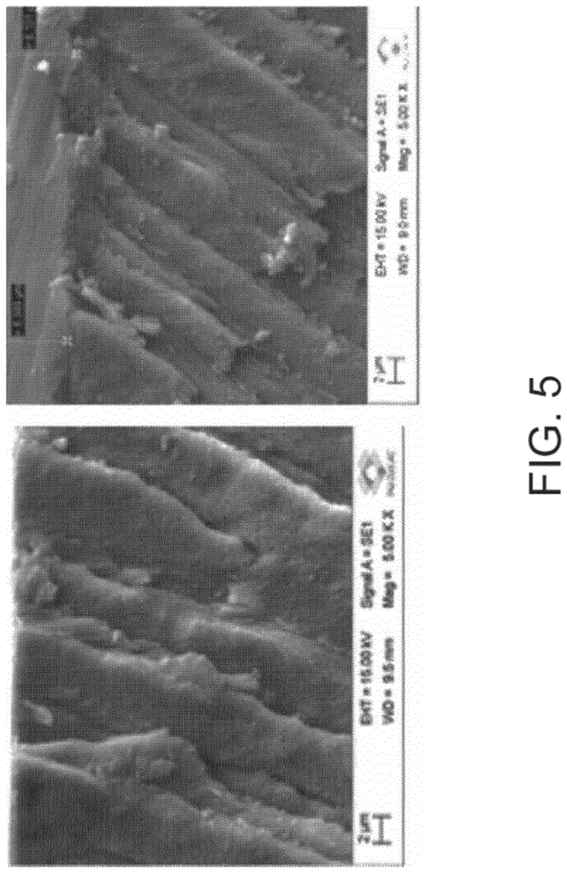

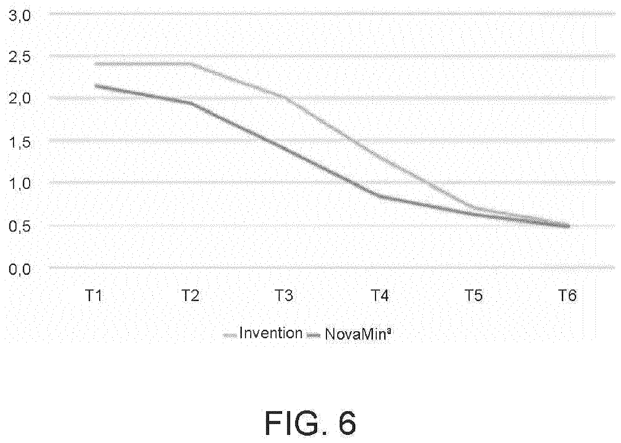

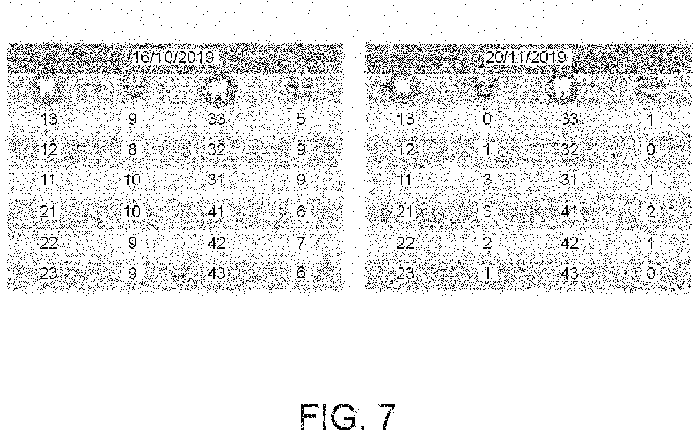

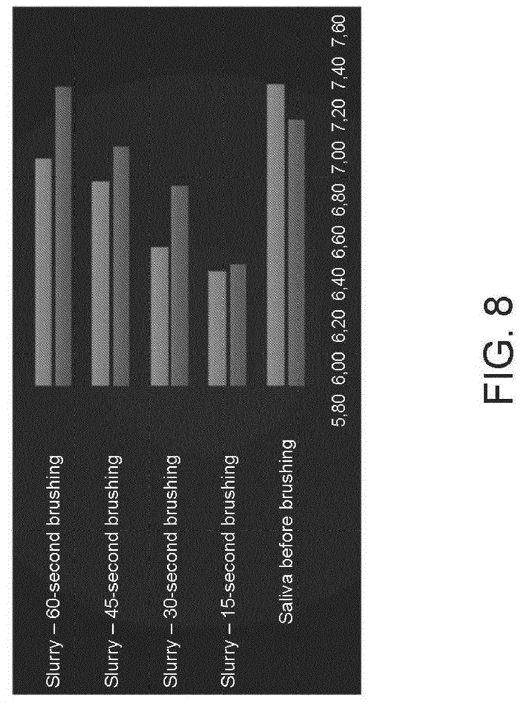

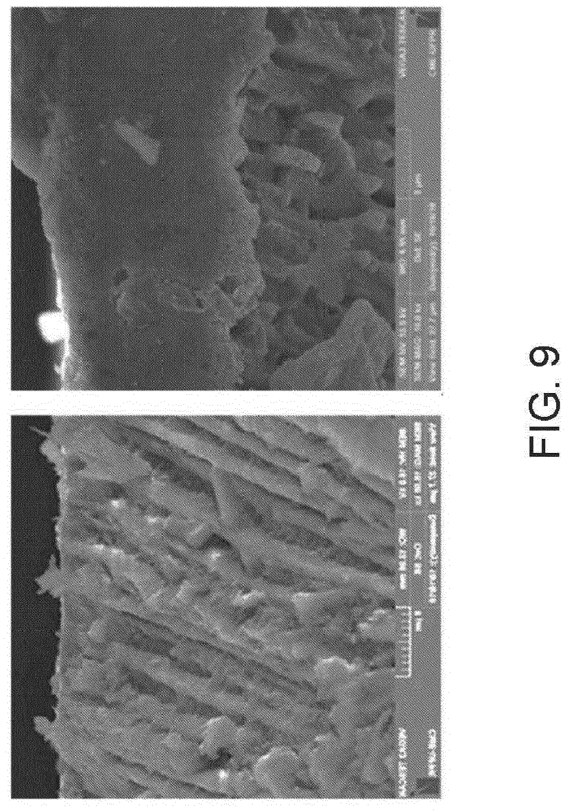

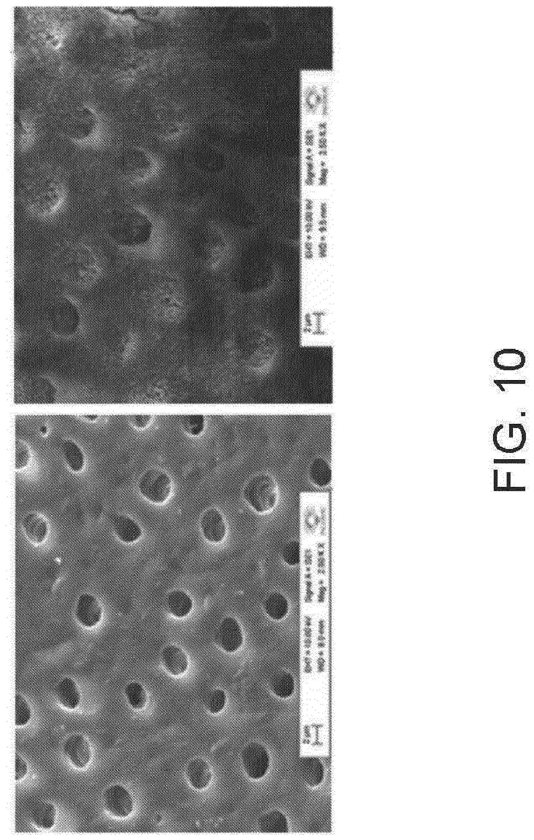

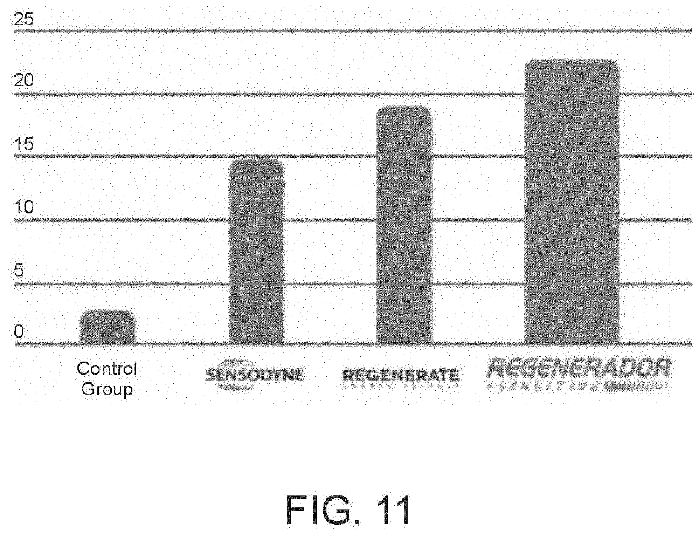

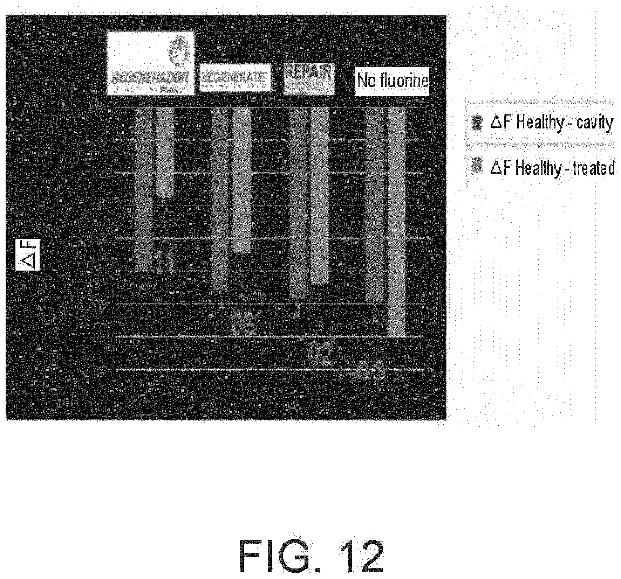

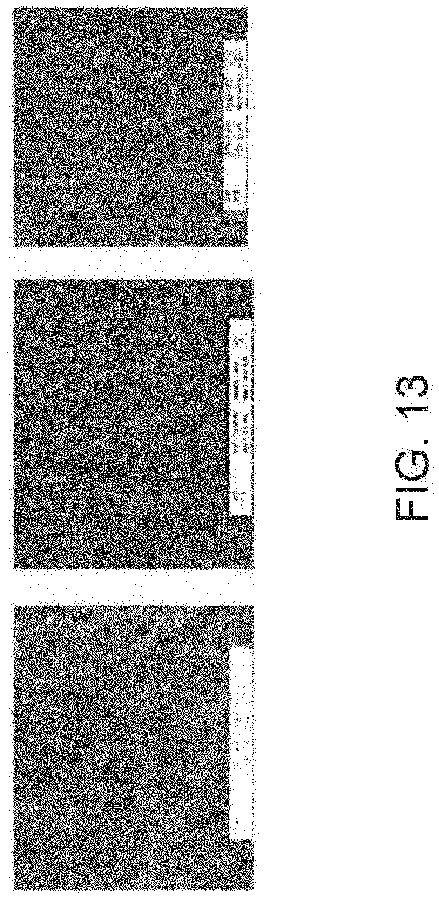

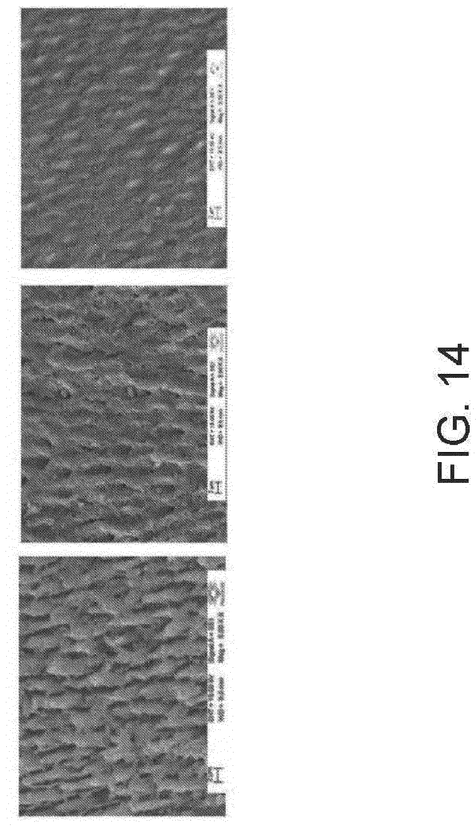

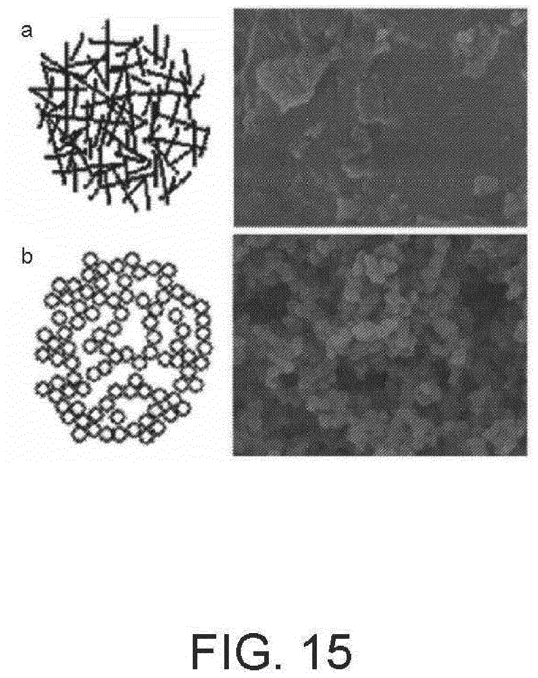

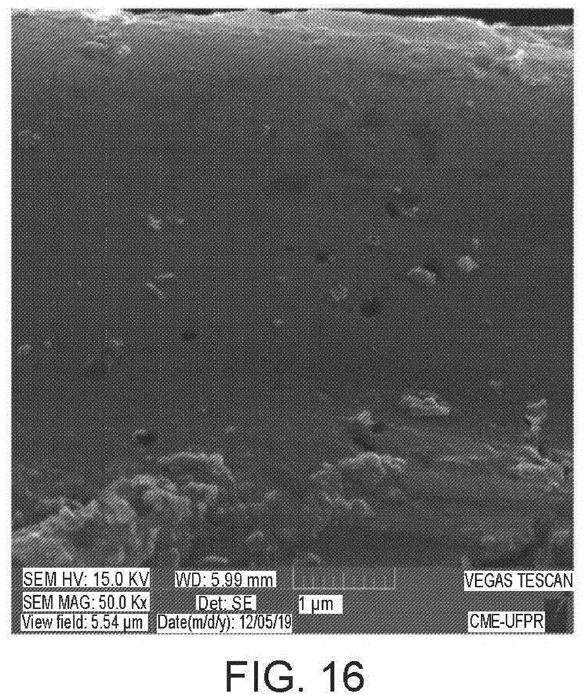

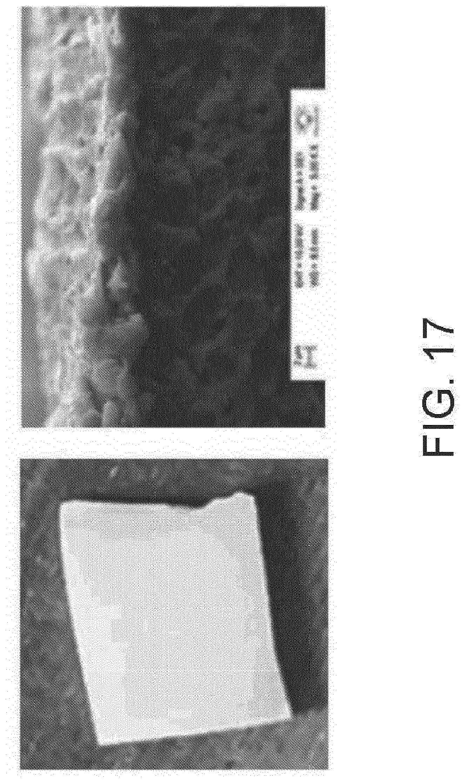

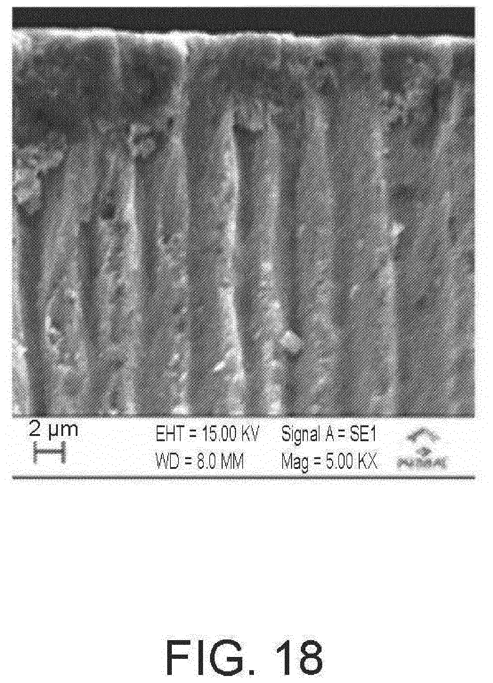

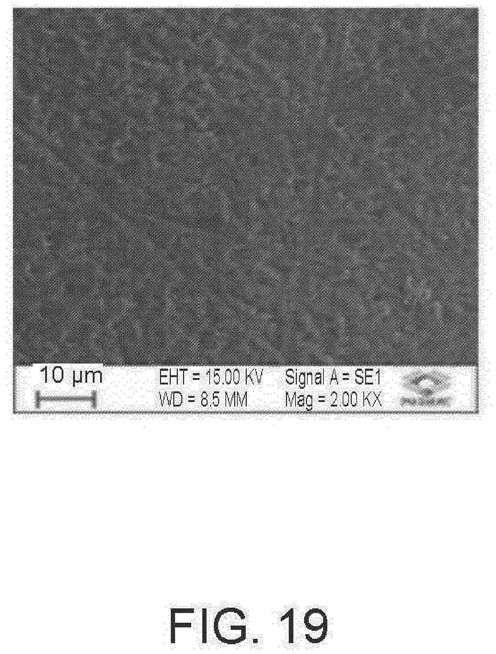

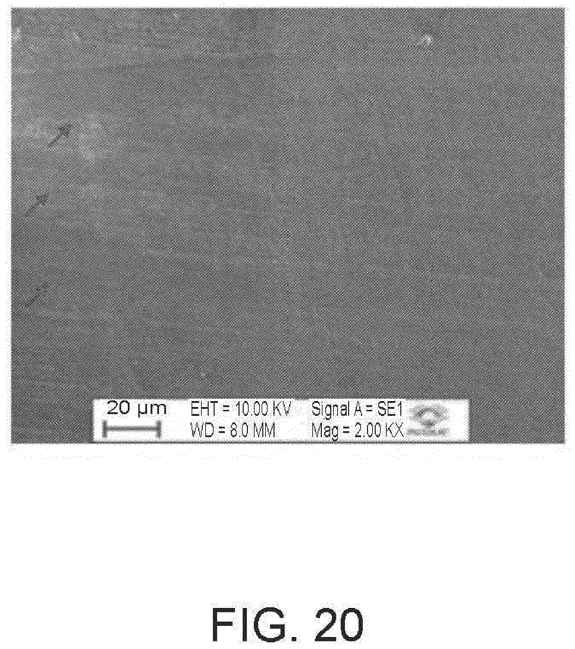

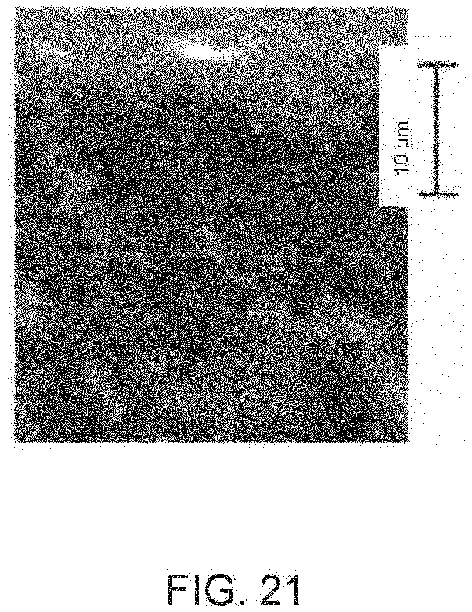

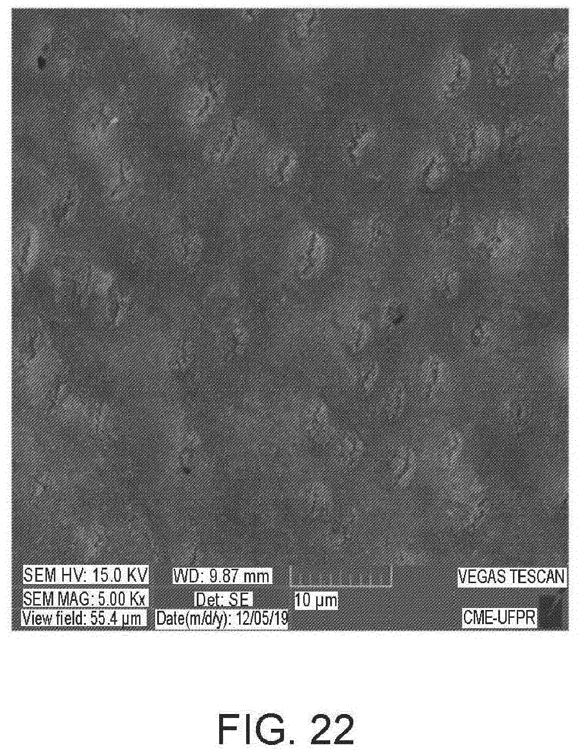

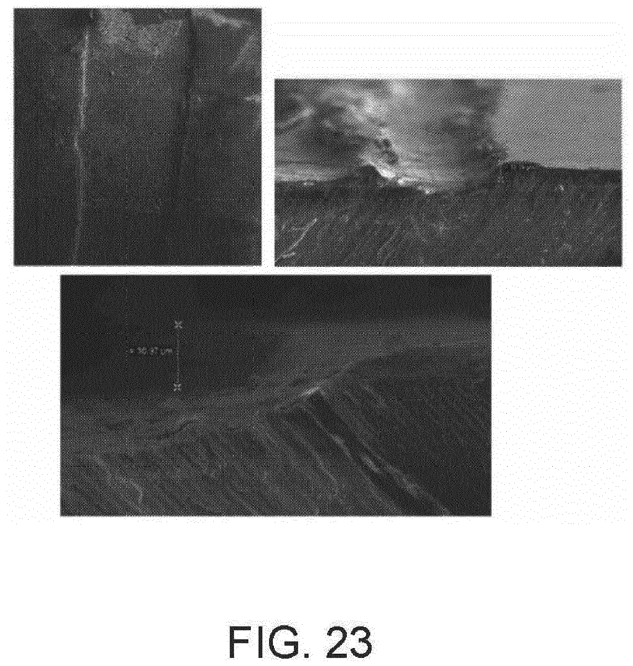

The invention will be described in a preferred embodiment; thus, for better understanding, references will be made to the following figures:
✔ FIG 1: A. EDS results of the composition of the invention, note the formation of the complex pattern. B. Enlarged section of the actual image, where: Strong purple - sodium; Violet - fluorine; Green-oxygen; Blue - silicon; Red - carbon; Yellow - phosphorus;
✔ FIG 2: EDS results of the use of the composition of the invention. A. Actual image; B. Enlarged image. Note the formation of the mineral pattern - calcium (blue) phosphate (pink), enriched with silicon (yellow);
✔ FIG 3: Image demonstrating the formation of the hybrid layer, *in situ;*
✔ FIG 4: Images of the process taking place inside the tooth-inside dentin tubules, illustrating the closure of the tubules;
✔ FIG 5: Enamel surface without applying the invention (left) and enamel surface brushed with the composition pleaded for one week;
✔ FIG 6: Illustrative chart of pain reduction from use of the oral composition and the Novamin^{®} technology;
✔ FIG 7: Scheme illustrating the visual analog pain scale (EVA);
✔ FIG 8: Illustrative chart of pH variation in relation to use;
✔ FIG 9: Cross-section of enamel showing the area without brushing This invention, and the area brushed with This invention, respectively;
✔ FIG 10: Images of the surface of tooth samples: Sound dentin open tubules simulating pain by dentin sensitivity and dentin treated with This invention, respectively;
✔ FIG 11: Comparison of the percentage of remineralization (% SMHR) of dentifrices between groups (Different letters indicate statistically significant difference between groups, ANOVA, P < 0.01);
✔ FIG 12: Absolute values and standard deviation of sound lesion depth for cavity and sound for treated cavity (Different letters indicate statistically significant between groups, ANOVA, P < 0.01);
✔ FIG 13: Images of the surface of tooth samples: sound enamel, enamel treated with MCBS, enamel treated with This invention, respectively;
✔ FIG 14: Images of the surface of tooth samples: sound enamel, enamel treated with regenerative agent, enamel treated with This invention, respectively;
✔ FIG 15: Images of Scanning Electron Microscopy of silica-based, lab-synthesized hybrids, with up to 60,000x amplification. A. synthesized in acidic medium; B. synthesized in basic medium;
✔ FIG 16: Image of tooth sample depth with highlight to the formed layer. Note that the scale is 1 micrometer, divided into 10 parts (100 nanometers each), not possible to notice any pores, only holes due to the displacement of material during cutting;
✔ FIG 17: Image that demonstrates formation of layers, both on dentin and on enamel (B). After the brushing period, a blue whitening tone was verified (A);
✔ FIG 18: Cross-sectional image showing tooth sample depth with highlight to the formed layer. Note a layer that varies between 4 and 8 micrometers with only four applications of 5 minutes of the pleaded composition in 2 phases, one containing the original formulation and another adding the entry calcium supplement - 6% calcium glycerophosphate;
✔ FIG 19: Image of enamel surface treated with This invention in 2 phases, one containing the original formula and another adding 2 sources of extra nanoparticulated calcium supplement (tricalcium phosphate and 5% calcium carbonate + F. Detail for filling of grooves by layer formation. This embossing thus favors retention of adhesive restorations/cementum. Result obtained from four applications;
✔ FIG 20: Image of enamel surface treated with the composition described in this invention for one week. Half of the sample was not brushed where it is possible to show the grooves (left side). On the brushed side, the grooves were filled with the mineral layer, showing the regenerative effect of This invention;
✔ FIG 21: Image of cross section of treated dentin with the composition described in This invention in 2 phases, one containing the original formula and another adding 2 sources of extra nanoparticulated calcium supplement (tricalcium phosphate and 5% calcium carbonate). Detail of tubule filling through a robust layer formation (up to 10 micrometers). Without calcium, a layer up to 3 micrometers was attained. Result with four application of 05 minutes; ✔ FIG 22: Image of dentin surface treated with This invention without fluorine. Detail of tubule filling;
✔ FIG 23: Images of the construction of a new enamel-like layer after one week of use. Images above show the worn tooth without using the technology, image below show the brushing and reconstruction of mineral loss, with around 40% mineral recovered after one week of use.

### DETAILED DESCRIPTION OF THE INVENTION

This invention refers to a composition in three variations, provided in a single phase or phases separated by physical barriers, inside a single container or encapsulated, or yet in separated recipients with components included in smaller sizes or in nanometric scale. The presentation form may be, but not limited to dentifrice (cream, gel, foam, liquid, or powder), mouthwash, drops and chewing gums. Below are the proposed variations, application examples, as well as its processes for obtaining:
✔ Variation 1: Variation for professional use, comprising phases 1, 2 and 3, with 0.01 to 30% of phase 3 + 0.5 to 40% of phase 2 + 30 to 60% of phase 1;
✔ Variation 2: Variation for domestic and professional use, comprising phases 1 and 2, with 0.5 to 70% of phase 2 + 30 to 99.5% of phase 1;
✔ Variation 3: Variation of domestic and daily use, comprising only phase 1, with 100% of phase 1.

For the application of variation 1, the dentist, while in possession of the three phases, shall start application by dispensing phase 3 directly onto the tooth through a syringe with applicator, wait 10 to 20 seconds and subsequently washing the tooth surface with water for 20 to 60 seconds. Afterwards, phases 2 and 1 shall be applied, previously mixed in the doctor's office, at a ratio that may vary between 1/2, 1/1, 2/1, not limited to said ratios, directly on the tooth with the help of a spatula or other application instrument. For application of variation 2, the patient opens the recipient containing phase 2 and spreads it over the toothbrush, in an amount the size of a pea. Subsequently repeat the process with the recipient containing phase 1. Immediately after application of both phases to the toothbrush, brushing is to be carried for 1 to 3 minutes, expelling foam and all excess material; mouth rinsing is not required. For application of variation 3, the patient opens the recipient and spreads its contents over the toothbrush, in an amount the size of a pea. Immediately after application of both phases to the toothbrush, brushing is to be carried for 1 to 3 minutes, expelling foam and all excess material; mouth rinsing is not required. In one application form, the oral composition with synergistic association of organic and inorganic components may comprise three phases, which are physically separated by layers, and/or encapsulation, and/or yet in distinct packaging, so that components do not interact before the activity time in the mouth, in other words, components are not neutralized becoming inactive:
✔ Phase I: main base composition, with or without fluorine, containing an acidified bioactive complex, with or without pigments, dyes, silicon sources, with or without whitening effect;
✔ Phase II: mixture of organic and/or inorganic accelerator such as calcium (micro and/or nanoparticles); and/or other agents, such as: antimicrobial agent, remineralizer (arginine), bleacher; phase with acidulated to slightly alkaline pH, struck by the addition of acids and/or phosphates or even other pH regulating agents;
✔ Phase III: acidified gel and thickened with silica, pH 2 to 4.

Considering that variation 3 of the composition, presented in a single recipient, must be physically separated by an inert layer, for example, of glycerin or a single composition with or without active fluorine; or, yet, due to encapsulation of phases 1 and 2, which will be dispersed in an inert matrix, with or without active fluorine.

In order to obtain the composition, components may or may not be in the nanometric scale, considering that, initially, all components of the composition are separated and weighed, according to table 4, as follows:

**Table 4: Components used to obtain the oral composition.**

| Components | Quantity % |
|---|---|
| Moistener | 40 to 70 |
| (Selected among, but not limited to: PEG 600, PEG 400, glycerin, Sorbitol in isolation or in association). | |
| Thickener | 5 to 30 |
| (Selected among, but not limited to: carboxymethylcellulose, xanthan gum, thickening silica, in isolation or in association). | |
| Deionized Water | 0.1 to 7 |
| Fluorides | 0 to 1 |
| (Selected among, but not limited to: stannous fluoride, sodium fluoride, potassium fluoride, sodium monofluorophosphate, sodium fluorosilicate, ammonium fluorosilicate, amine fluoride (for example, N'-octadecyltrimethylendiamine-N, N, N'-tris (2- ethanol) -dihydrofluoride), ammonium fluoride, titanium fluoride, hexafluorosulfate and combinations thereof). | |
| Sweeteners | 0.5 to 5 |
| (Selected among, but not limited to: sodium saccharine and xylitol). | |
| Preservatives (Selected among, but not limited to sodium benzoate, methylparabens, parabens) | 0.1 to 1 |
| Remineralizing salts, desensitizers, catalysts (Selected among, but not limited to: sodium ions, calcium, potassium, iron, zinc, tin, magnesium, titanium, aluminum and/or copper) | 0.1 to 10 |
| Abrasive (Selected among, but not limited to: calcium carbonate, sodium, silica). | 3 to 18 |
| Surfactant | 5 to 15 |
| (Selected among, but not limited to: sodium laureth sulfate, sodium alkyl sulfate, sodium lauroyl sarcosinate, cocamidopropyl betaine and polysorbate and combinations thereof. Preferably, sodium laureth sulfate) . | |
| Antiseptic | 0.1 to 1 |
| (Selected among, but not limited to: halogenated diphenyl ether, triclosan, herb extracts, essential oils, rosemary extract, tea extract, magnolia extract, thymol, menthol, eucalyptol, geraniol, carvacrol, citral, hinokitol, catechol, methyl salicylate, epigallocatechm gallate, epigallocatechm, gallic acid, miswak, sea-buckthorn extract, biguanide antiseptics, chlorhexidine, alexidine or octenidine, quaternary ammonium composites, cetylpyridinium chloride (CPC), benzalkonium chloride, tetradecyl pyridinium chloride (TPC), N-tetradecyl-4-ethylpyridinol chloride, N-tetradecyl-4-ethylpyridinol chloride, octenidine, sanguinarine, Povidone-iodine, delmopinol, salifluorine, tin salts, copper salts, iron salts, sanguinarine, propolis and oxigenating agents, hydrogen peroxide, buffered sodium peroxoborate or peroxocarbonate, phthalic acid and its salts, monopertallic acids and its salts and esters, ascorbyl stearate, oleoylsarcosine, alkyl sulfate, dioctyl sulfossuccinate sulfate, salicylanilide, domiphen bromide, delmopinol, octapinol and other piperidine byproducts, nicin preparations, chlorite salts or any mixture thereof). | |
| Flavoring agent (Selected among, but not limited to: essential oils (mint, peppermint, spearmint, lemon grass, clove, salvia, strawberry, grape, eucalyptus, marjoram, cinnamon, lemon, rosemary - pepper, orange), as well as aldehydes, esters, alcohols and similar flavoring materials). | 1 to 5 |
| Dyes/pigments | 0.1 to 10 |
| (May be organic or inorganic, selected among, but not limited to: peroxides, superoxides, oxygen forming agents and ingredients for optical bleaching as all dyes and pigments, organic and inorganic, which act within the blue to violet light spectrum to reflect white light, such as mica and silicon compounds). | |
| pH corrector | 0.5 to 40 |
| (Selected among, but not limited to: basic (sodium mono- and di-phosphates) and acids (phosphoric, citric, maleic)) . | |
| Calcium Sources | 0.001 to 10 |
| (Which are alternatively employed and selected among, but not limited to: calcium glycerophosphate, calcium carbonate and tricalcium phosphate, may be organic sources or not). | |
| Silicon source | 0.1 to 40 |
| (Which are alternatively employed and selected among, but not limited to: amorphous silica, bioglasses, silicates, mesoporous silica, functionalized silica, especially developed silica). | |
| Amino acids | 0 to 10 |
| (Which are alternatively employed and selected among, but not limited to: arginine, lysine, citrulline, ornithine, creatine, histidine, diaminobutanoic acid, diaminopropionic acid, salts and/or combinations thereof, or even any amino acids with a carboxyl group and a water-soluble amino group and available in aqueous solution with a pH around 7 or lower. Preferably, arginine). | |
| Orthophosphoric acid | 0 to 40 |
| Tetrasodium pyrophosphate | 0.5 to 40 |

Below are some examples of formulations and the process for obtaining each of the phases that comprise the three variations of the pleaded composition.

Table 5 below describes an example of formulation of phase 1 of the composition, and the process for obtaining it.

**Table 5: Components present in the formulation of phase 1.**

| Component | Proportion |
|---|---|
| Moisteners (PEG 600, Glycerin, Sorbitol) | 40 to 70 |
| Thickener (Carboxymethylcellulose) | 1 to 5 |
| Deionized Water | 0.1 to 30 |
| Sweeteners (Sodium saccharine, xylitol) | 0.1 to 1 |
| Sodium Fluoride | 0 to 1% |
| Preservative (Sodium Benzoate) | 0.1 to 1 |
| Silica Thickener | 7 to 15 |
| Abrasive Silica | 7 to 15 |
| Tetrasodium pyrophosphate | 0.5 to 40% |
| Surfactant (Sodium laureth sulfate) | 3 |
| Aroma | 0.5 to 3 |
| Antiseptic (Triclosan) | 0.1 to 0.3 |
| Orthophosphoric acid | 0.8 to 1.5 |

In order to obtain the formulation of phase 1 of the composition, a pre-mix is prepared, where the glycerin + CMC base is obtained, and for such the following components are used:
✔ Carboxymethylcellulose, at a 0.7 to 1.0% m/m proportion of the formulation;
✔ Glycerin, at a 45 to 55% m/m proportion of the formulation.

Carboxymethylcellulose is added to glycerin, in slow agitation, for 10 to 30 minutes at a speed of 45,000 to 200,000 rpm at 25°C, to avoid formation of lumps, obtaining the glycerin + CMC base. Afterwards, the full load of deionized water is added to the reactor, at 0.1 to 7% regarding the total volume of the composition, activating turbine, anchor, and impeller. Then the following salts are added to the reactor: sodium fluoride at a 0 to 1% m/m proportion of the formulation; sodium saccharine at a 0.5 to 5% m/m proportion of the formulation; sodium benzoate at a 0.1 to 1% m/m proportion of the formulation; xylitol at a 0.5 to 5% m/m of formulation; tetrasodium pyrophosphate at a 0.5 to 40% m/m of the formulation, followed by homogenization for 5 to 20 minutes in the conditions previously described.

Afterwards, moisteners and sorbitol are added at a 40 to 70% m/m proportion of the formulation, and PEG-600 at a 40 to 70% m/m proportion of the formulation, and the previously prepared glycerin + CMC base at a 50 to 55% m/m proportion of the formulation. A vacuum application is then performed at 600 mmHg, followed by homogenization for 5 to 20 minutes in the conditions described above. After homogenization with the vacuum turned on, the thickener silica is slowly added at a 10-to-15-minute interval at a 7 to 15% m/m proportion of the formulation; then the abrasive silica is added the same way, at a 7 to 15% m/m proportion of the formulation. With the vacuum still on, homogenization is carried out for a period of 1 to 3 hours.

After this period, with the vacuum still on, the sodium laureth sulfate is added at a 5 to 15% m/m proportion of the formulation; followed by homogenization for a period of 5 to 20 minutes. Then triclosan is added at a 0.1 to 1% m/m proportion of the formulation, previously dissolved in menthol at a 1 to 5% m/m proportion of the formulation; followed by homogenization for a period of 5 to 20 minutes. Lastly, the orthophosphoric acid is added at a 0.8 to 1.5% m/m proportion of the formulation, also with the vacuum turned on, followed by homogenization for a period of 3 to 5 hours, after which the process for obtaining phase 1 is finished, which may be applied in isolation, as the variation 3 of the composition.

Table 6 below describes an example of formulation of phase 2 of the composition, which contains calcium, followed by the process for obtaining it.

**Table 6: Components present in the formulation of phase 2.**

| Component | Proportion |
|---|---|
| Moisteners | 40 to 70 |
| Thickener | 1 to 5 |
| Deionized Water | 0.1 to 30 |
| Sweeteners | 0.1 to 1 |
| Preservative | 0.1 to 1 |
| Abrasive | 7 to 15 |
| Calcium Source | 5 |
| (Microlyzed 70 to 80% calcium carbonate / 20 to 30% tricalcium phosphate) | |
| Surfactant | 3 |
| (Sodium laureth sulfate) | |
| Aroma | 0.5 to 3 |
| Antiseptic | 0.1 to 0.3 |
| pH corrector | 2 to 5 |
| (Monobasic Phosphate, dibasic Phosphate, citric acid) | |

In order to obtain the formulation of phase 2, initially, a pre-mix is prepared, where the glycerin + CMC base is obtained, and for such the following components are used:
✔ Carboxymethylcellulose, at a 0.7 to 1.0% m/m proportion of the formulation;
✔ Glycerin, at a 45 to 55% m/m proportion of the formulation.

Carboxymethylcellulose is added to glycerin, in slow agitation, for 10 to 30 minutes at a speed of 45,000 to 200,000 rpm at 25°C, to avoid formation of lumps, obtaining the glycerin + CMC base. Afterwards, the full load of deionized water is added to the reactor, at 0.1 to 7% m/m proportion of the formulation, activating turbine, anchor, and impeller. Then the following salts are added to the reactor: sodium saccharine at a 0.5 to 5% m/m proportion of the formulation; sodium benzoate at a 0.1 to 1% m/m proportion of the formulation; xylitol at a 0.5 to 5% m/m proportion of the formulation; citric acid at a 2 to 5% m/m proportion of the formulation, followed by homogenization for 5 to 20 minutes in the conditions previously described. Afterwards, moisteners and sorbitol are added at a 40 to 70 m/m proportion of the formulation, and PEG-600 at a 40 to 70 m/m proportion of the formulation, and the previously prepared glycerin + CMC base at a 50 to 55% m/m proportion of the formulation. A vacuum application is then performed at 600 mmHg, followed by homogenization for 5 to 20 minutes in the conditions described above. After homogenization with the vacuum turned on, the thickener silica is slowly added at a 10-to-15-minute interval at a 7 to 15% m/m proportion of the formulation, and the mixture of 70% to 80% calcium carbonate and 20 to 30% microlyzed tricalcium phosphate is added at a 0.1 to 5% m/m proportion of the formulation. With the vacuum still on, homogenization is carried out for a period of 1 to 3 hours. After this period, with the vacuum still on, the sodium laureth sulfate is added at a 5 to 15% m/m proportion of the formulation; followed by homogenization for a period of 5 to 20 minutes. Then triclosan is added at a 0.1 to 1% m/m proportion of the formulation, previously dissolved in menthol at a 0.5 to 3% m/m proportion of the formulation; followed by homogenization for a period of 5 to 20 minutes. Lastly, the pH corrector is added, which may be monobasic phosphate, dibasic phosphate or citric acid, as needed, at about 2 to 5% m/m proportion of the formulation, also with the vacuum on, to adjust around a pH of 4.5 to 5.5 followed by homogenization for a period of 0.5 to 1 hour, after which the process for obtaining phase 2 is finished, which may be applied in association with phase 1, as a variation 2 of the composition, or in association to phases 1 and 2, as variation 1.

Table 7 below describes an example of formulation of phase 3 of the composition, which contains calcium, followed by the process for obtaining it.

**Table 7: Components present in the formulation of phase 3.**

| Component | Proportion |
|---|---|
| Moisteners (PEG 600, Glycerin, Sorbitol) | 40 to 70 |
| Thickener (Carboxymethylcellulose) | 1 to 5 |
| Deionized Water | 0.1 to 30 |
| Abrasive (Silica Thickener) | 7 to 15 |
| Blue dye | 0.1 to 0.3 |
| Citric acid | 5 to 40 |

In order to obtain phase 3, wherein it is composed of a phosphoric acid gel, a pre-mix is prepared, where the glycerin + CMC base is obtained, and for such the following components are used:
✔ Carboxymethylcellulose, at a 0.7 to 1.0% m/m proportion of the formulation;
✔ Glycerin, at a 45 to 55% m/m proportion of the formulation.

Carboxymethylcellulose is added to glycerin, in slow agitation, for 10 to 30 minutes at a speed of 45,000 to 200,000 rpm at 25°C, to avoid formation of lumps, obtaining the glycerin + CMC base. Afterwards, the full load of deionized water is added to the reactor, at 0.1 to 7% m/m proportion of the formulation, activating turbine, anchor, and impeller. After homogenization with the vacuum turned on, the thickener silica is slowly added in a 10-to-15-minute interval at a 5 to 25% m/m proportion of the formulation, then the blue dye, at a 0.1 to 0.3% m/m proportion of the formulation. With the vacuum still on, homogenization is carried out for a period of 1 to 2 hours. Lastly, the citric acid is added at a 5 to 40% m/m proportion of the formulation, also with the vacuum turned on, followed by homogenization for a period of 0.5 to 1 hour, after which phase 3 is obtained, which may be applied by a professional, in association with phases 1 and 2, and as variation 1 of the composition.

Table 8 below shows a summary board with the characteristics of the pleaded oral composition, in any one of its three variations.

**Table 8: Characteristics of the oral composition obtained, in any of its variations.**

| | Proposed oral composition |
|---|---|
| In tube (Formulation) | -- Water |
| | ++ Acidic |
| | + Phosphate |
| | +Na, K, Zn |
| | + Silica |
| | ± Fluorine |
| | Silicon-based complex |
| | pH 4.7 - acidified at pH 2.0 to 5.5 |
| | |
| In mouth | + Phosphate |
| | pH 5.5 to 6.5 |
| | ++ FCa2 Phosphate |
| | ++Hybrid Layer: Si, P, Ca, proteins (with or without fluorine) |
| | |
| On tooth | ++ Enamel permeability / ++ Ca availability |
| | + Silicon-based complex |
| | ++ Mineral precipitation |
| | Hybrid Layer: Si, P, Ca, proteins (with or without fluorine) |
| | Formation of microporous layer - acidic medium |
| | Active layer (molecule release and adhesion) |
| | |
| Results | + Remineralization |
| | + Hybrid layer (protection/adhesion) |
| | + Hybrid layer anti-dental erosion |
| | + Antimicrobial |
| | Whitening |
| | + Desensitization |

In this description, except otherwise provided, the use of the singular form also includes the plural. For example, "a sodium ion source" also comprises the case in which more than one source of sodium ions may be used, in addition to its complex or ionized form. The term "non-aqueous" referred herein in this invention relates to the use of small amounts of water, or no water at all, in its formulation; this explanation is required in order to distinguish the term aqueous (oral environment) where the invention operates. Examples of formulations described for each one of the phases are illustrative and non-limiting, and such formulations may eventually feature other components, such as fluorine. In addition, it should be emphasized that any of the three variations may still, alternatively, be supplemented with calcium salts, using up a proportion of 0.001 to 10% m/m of the composition, preferably with calcium glycerophosphate, calcium carbonate and tricalcium phosphate. The oral composition described herein follows the process for obtaining silicon-based polymers in its structure, in acidic medium and with little to no water. It refers to the use of all compounds containing silicon, preferably all forms of silica and, preferably, among silicas, amorphous silica, due to its low cost. The process is considered low cost, since other more expensive processes and silicon compounds are used in the state of the art, such as: bioglasses, silicates, mesoporous silica, functionalized silica, especially developed silica, among others. Obtaining these oral compositions, silicon-based complexes, happens through addition, or not, of metallic salts during preparation, as well as organic compounds that are part of the formulation. Through an EDS (Energy-Dispersive Spectroscopy) mapping analysis (Fig. 1) it is possible to notice the formation of the silicon-based complex. Immediately after the variations come into contact with the oral cavity (aqueous medium), their action starts. In further detail, the reaction takes place inside the dental structure, where, when applying This invention, acidification of the medium and the tooth is provoked, causing an acidic attack which releases tooth components, more specifically calcium and phosphates. The silicon-based complex is electronically attracted by the tooth surface, adhering to it and promoting increase of the polymeric chain, capturing negative and positive ions, as well as organic and inorganic molecules. This "capturing" process, mainly for calcium, increases the interface pH to alkaline levels, favoring the "maturing" of calcium phosphates and, consequently, mineral condensation and formation. This "maturing" of calcium phosphates is known by the formation of hydroxyapatite, which is confirmed by the Ca/P relation in the EDS around 1.64 at the atomic number, very close to optimal at 1.67. However, the formed hydroxyapatite is not pure; otherwise, it would be soluble in acidogenic challenges with a pH around 5.5. Thus, the formation of an insoluble complex occurs in This invention, enriched with silicon and maybe containing fluorine (Fig. 2). The continuous use of This invention promotes, on the dental structure (*in situ)* the formation of an increasingly robust, active, compact, low porosity layer, resistant to the challenges of the harmful effects caused to teeth (Fig. 3). This is only possible because continuous formation of the layer only takes place due to "polishing/preparation" of the layer that has been deposited previously. The reaction is caused by rubbing the composition over the already existing structure, in other words, through application of the composition that is acidified and highly reactive, with the help of a toothbrush or other means. Basicity is higher between the tooth and the formed layer, and the medium precipitates the layer even further due to its acidity. This probably occurs because the free calcium present on the tooth and saliva accelerates polymerization/condensation of the layer, in addition to increasing pH on the interface.

As the layer condensation depends on calcium and phosphates available in the medium, the main source of these components in the buccal medium is the enamel. In this sense, higher layer thicknesses were found about this tissue (around 6 to 20 micrometers). The dentin, on the other hand, layers up to 3 micrometers were found before the use of the pleaded composition. The lack of supplementation for layer formation on the dentin was overcome by the supplementation of calcium and phosphate layers. Calcium and phosphate salts were added in the composition during preparation of phase II, so that the compounds would not react before application. The result was the increase in layer thickness to around 6 to 10 micrometers (Fig. 5). As the composition is used, a hybrid layer is formed with bonding capacity for organic/inorganic layers, which are released only if needed. These components are comprised by antimicrobial agents, boosters, remineralizers and desensitizers. The adhesion to the organic part of the layer is given by the carbon-bearing compounds, which are incorporated through formula ingredients, among which are PEG, and may also be bonded to surfactants (for example, sodium laureth sulfate), responsible for providing elasticity and stability.

Below are some trials carried out with the oral composition (C) described above and pleaded in This patent application.

### CLINICAL TRIALS

A randomized double-blind clinical trial was carried out to evaluate the capacity of pain relief on a patient with dentin sensitivity and need for periodontal treatment. Where T1: pain before the periodontal treatment; T2: after periodontal treatment; T3: 1 week of use of the oral composition (C); T4: 2 weeks of use; T5: 3 weeks of use, and; T6: 4 weeks of use; preliminary results did not yield statistically significant differences between the groups, however, as the pain level of the sample that used This invention started higher and ended up equal to the patients that used the product Novamin^{®}, it may be stated that This invention was capable of reducing pain further than the commercially available product (Fig. 6). A 12-year-old child suffering from MIH - Molar Incisor Hypomineralization, reporting severe pain caused by dental sensitivity, used the product Elmex Sensitive^{®} for 30 days and experienced pain reduction from a 10 to a 7, in a pain scale from 0 to 10. The same child began using This invention and, after 30 days of use, its average initial pain level dropped from 8 to 1, as per Figure 7. A pH analysis was carried out to verify the behavior of compositions throughout the use, both for the oral composition and another composition of similar acidic pH were used. In the study, patients used gels and took notes of pH variation during teeth brushing. Results obtained are observed in figure 8, where blue shows that the gel described in the prior art BR102013006807-1, with acidic pH at 4.5, does not remain acid for extended periods, and is rapidly neutralized by saliva. The orange bar, on the other hand, demonstrates that the use of the oral composition (C), described in This invention, had a progressive increase in pH, ending slightly acidic with 1 minute, still below baseline data. The result obtained is highly desired, in view of the polymer precipitation reaction followed by subsequent condensation and formation of hybrid layer, takes place in an aqueous and acidic medium.

### LABORATORY STUDIES

Bovine enamel blocks were prepared and demineralized. Half a portion of each was then covered with enamel and the exposed area was brushed with the oral composition, described in This invention. Analysis of Scanning Electron Microscopy - SEM and Energy-Dispersion Spectroscopy - EDS (Fig. 8) have validated the deposition and composition of the hybrid layer, formed by the oral composition described. Tables 8 and 9 show the composition of enamels before and after application of the oral composition. The same study was carried out using bovine dentin (Fig. 9), demonstrating the same effect.

**Table 8: EDS - Demineralized Enamel.**

| Element | %Weight | % of atomic number |
|---|---|---|
| C | 18.17 | 26.77 |
| He | 53.58 | 59.26 |
| Na | 0.60 | 0.46 |
| Mg | 0.22 | 0.16 |
| P | 9.61 | 5.49 |
| Ca | 17.82 | 7.87 |
| Total: | 100.00 | 100.00 |

**Table 9: EDS- Enamel brushed with the oral composition (C)-composition of hybrid layer.**

| Element | %Weight | % of atomic number |
|---|---|---|
| C | 39.31 | 54.14 |
| He | 31.09 | 32.14 |
| Na | 0.62 | 0.45 |
| Mg | 0.29 | 0.20 |
| Al | 0.38 | 0.24 |
| Si | 0.68 | 0.40 |
| P | 8.38 | 4.48 |
| S | 0.37 | 0.19 |
| Cl | 0.40 | 0.19 |
| K | 0.33 | 0.14 |
| Ca | 17.84 | 7.36 |
| Fe | 0.29 | 0.09 |
| Total: | 100.00 | 100.00 |

In another study, 10 blocks of bovine enamel previously prepared and demineralized for each of the groups (Regenerate^{®}, Sensodyne Repair^{®} in Protect, Colgate Reparação Diária^{®} and the oral composition (C), described in This invention) simulated the mouth environment for 1 week.

During the experiment, the surface hardness level of blocks before demineralization (baseline) was measured, and afterwards the blocks were demineralized, and a new hardness measurement was carried out. After the second measurement the blocks were brushed for a week in the brushing machine with each of the toothpastes. After this process was finished, a third hardness measurement was carried out after the blocks were brushed. The oral composition, described in This invention, was vastly superior to other toothpastes regarding remineralization levels in only 1 week, as observed in figure 10. A second measurement was performed with the QLF device which quantifies minerals through light. Results have also shown a large remineralization of demineralized dental tissues for the oral composition, still greatly superior to other toothpastes, recovering around 50% of the lost minerals in only 1 week of use (Fig. 12). In another study, with unique results in the state of the art, the protection capacity of dental enamel was evaluated. The mesoporous calcium silicate biomaterial-based toothpaste was used for one week (Regenerate^{®} dental gel) and the oral composition, described in This invention. Both products formed a protective layer at the end of one week of use (Fig. 13). After treatment, the samples were immersed in citric acid at 50% for 2 minutes, in order to simulate the dental erosion process. This erosion challenge promoted by citric acid is the same acid found in lemon juices, oranges, etc. The process was validated, since the sound dental enamel clearly was worn similarly to erosion, after the challenge. The enamel treated with the Regenerate^{®} dental cream attained a limited protection of enamel structure, possibly verified through Figure 14, where it clearly did not withstand the challenge.

Differently than what occurred with the dental enamel brushed with the oral composition (C) described in This invention, where practically no changes to the layer structure were observed, and the enamel below it remained intact (Fig. 14). The hybrid layer (Fig. 15A) formed by This invention, is a thicker and higher layer, more resistant, unique, formed in acidic medium, microporous, and hard to penetrate. Differently from the enamel treated with Regenerate^{®}, which formed a mesoporous layer (Fig. 15B), with larger diameter pores, with less thickness and smaller, which probably enabled the penetration of acid and degradation of said layer and the enamel. Therefore, gelation in acidic medium leads to chains that join to form polymeric gels (Fig. 16), that after drying give rise to a compact matrix with low pore volume and size generally smaller than 2 nm of diameter, called micropores (Benvenutti, 2009). In another study, bovine enamel and dentin blocks were prepared and brushed for 1 week with This invention, with while silica substituted by blue silica.

Layers formed both on the dentin and on the enamel. After the brushing period, a blueish tone and bleaching was verified (Fig. 17A). It was possible to verify the clarifying effect caused to the tooth through the spectrophotometer, confirming the positive result. Thus, it is possible to state, based on results, that a layer has formed, even if capable of providing an optical whitening effect (Fig. 17B). Figures 18 to 22 show the details of the hybrid layer formed with the use of the oral composition and figure 23 demonstrates the construction of a new enamel-like layer with one week of use of the oral composition.

This way, this invention is an acidified bioactive complex obtained from association of salts, organic compounds, components including silicon and phosphates. When the product is being used, in the mouth, it lowers the pH of the mouth cavity to around 5.5, due to its acidic composition. Thus, this invention creates an acidic condition in the dental structure to release mainly calcium and phosphate ions into the buccal medium, whilst the bioactive complex is electrochemically attracted by the tooth, bonds to it and gathers dispersed particles and ions, precipitating them and transforming into a hybrid layer containing silicon-enriched hydroxyapatite.

It should be highlighted that the hybrid layer may also be formed by all types of ions and calcium phosphate-based compounds, especially apatites. The hybrid layer remineralizes the dental enamel surface, functioning as a protective shield over the tooth, which mimics the original enamel, an "enamel-like" which functions against the acidic day-to-day challenges when the dentin is exposed, this layer obliterates the dentin tubules and relieves the pain caused by dentin sensitivity, and in addition, the composition is also a protective agent against cariogenic and microbial processes and exposure to acids even in pH below 4.5. The formation of a layer takes place in a self-etching manner, in other words, after each use of the oral composition variations, a new layer is formed on top of the previous one.

The inventive step of this application lies precisely in the formation of minerals in acidic medium, exactly in the same environment in which minerals are lost, for example, the solubilization of hydroxyapatite occurs in pH 5.5 or lower, enabling the formation of a hybrid layer, which allows the product to act upon full maintenance of oral health.

In face of the foregoing, it is observed that the oral composition with synergistic association of organic and inorganic components, its process for obtaining and uses thereof deserve the privilege of an invention patent.

## Claims

1. Oral composition with synergistic association of organic and inorganic components, wherein it features in its constitution, spread into three phases (phase 1, phase 2 and phase 3) and with the possibility of combination into three variations (variation 1, variation 2 and variation 3) the following components:
✔ Moistener: at a 40 to 70% m/m proportion of the composition; selected among PEG 600, PEG 400, glycerin, sorbitol, in isolation or combined;
✔ Thickener: at a 5 to 30% m/m proportion of the composition; selected among carboxymethylcellulose, xanthan gum, thickening silica, in isolation or combined;
✔ Deionized water: at a 0.1 to 7% m/m proportion of the composition;
✔ Fluorides: at a 0 to 1% m/m proportion of the composition; selected among: stannous fluoride, sodium fluoride, potassium fluoride, sodium monofluorophosphate, sodium fluorosilicate, ammonium fluorosilicate, amine fluoride (for example, N'-octadecyltrimethylendiamine-N, N, N'-tris (2- ethanol)-dihydrofluoride), ammonium fluoride, titanium fluoride, hexafluorosulfate and combinations thereof;
✔ Sweeteners: at a 0.5 to 5% m/m proportion of the composition; selected between sodium saccharine and xylitol;
✔ Preservative: at a 0.1 to 1% m/m proportion; selected among sodium Benzoate, methylparabens, parabens; preferably sodium Benzoate;
✔ Remineralizing salts, desensitizers and catalysts: at a 0.1 to 10% m/m proportion of the composition; selected among sidum, calcium, potassium, iron, zinc, tin, magnesium, titanium, aluminum and/or copper ions.
✔ Abrasive: at a 3 to 18% m/m proportion of the composition; selected between calcium carbonate, sodium, silica;
✔ Surfactant: at a 5 to 15% m/m proportion of the composition; selected among sodium laureth sulfate, sodium alkyl sulfate, sodium lauroyl sarcosinate, cocamidopropyl betaine and polysorbate and combinations thereof; preferably, sodium laureth sulfate;
✔ Antiseptic: at a 0.1 to 1% m/m proportion of the composition; selected among halogenated diphenyl ether, triclosan, herb extracts, essential oils, rosemary extract, tea extract, magnolia extract, thymol, menthol, eucalyptol, geraniol, carvacrol, citral, hinokitol, catechol, methyl salicylate, epigallocatechm gallate, epigallocatechm, gallic acid, miswak, sea-buckthorn extract, biguanide antiseptics, chlorhexidine, alexidine or octenidine, quaternary ammonium composites, cetylpyridinium chloride (CPC), benzalkonium chloride, tetradecyl pyridinium chloride (TPC), N-tetradecyl-4-ethylpyridinol chloride, N-tetradecyl-4-ethylpyridinol chloride, octenidine, sanguinarine, Povidone-iodine, delmopinol, salifluorine, tin salts, copper salts, iron salts, sanguinarine, propolis and oxigenating agents, hydrogen peroxide, buffered sodium peroxoborate or peroxocarbonate, phthalic acid and its salts, monopertallic acids and its salts and esters, ascorbyl stearate, oleoylsarcosine, alkyl sulfate, dioctyl sulfossuccinate sulfate, salicylanilide, domiphen bromide, delmopinol, octapinol and other piperidine byproducts, nicin preparations, chlorite salts or any mixtures between any aforementioned substances; preferably, triclosan;
✔ Flavoring agent: at a 1 to 5% m/m proportion of the composition, selected among essential oils (mint, peppermint, spearmint, lemon grass, clove, salvia, strawberry, grape, eucalyptus, marjoram, cinnamon, lemon, rosemary - pepper, orange), as well as aldehydes, esters, alcohols and similar flavoring materials;
✔ Pigments/dyes: at a 0.1 to 10% m/m proportion of the composition; may be organic or inorganic, selected among peroxides, superoxides, oxygen forming agents and ingredients for optical bleaching as all dyes and pigments, organic and inorganic, which act within the blue to violet light spectrum to reflect white light, such as mica and silicon compounds;
✔ pH corrector: at a 0.5 to 40% m/m proportion of the composition; selected among basics (mono- and di-sodium phosphates) and acids (phosphoric, citric, maleic);
✔ Calcium sources: at a 0.001 to 10% m/m proportion of the composition; selected among: calcium glycerophosphate, calcium carbonate and tricalcium phosphate, from organic sources or not;
✔ Amino acids: at a 0 a 10% m/m proportion of the composition; selected among arginine, lysine, citrulline, ornithine, creatine, histidine, diaminobutanoic acid, diaminopropionic acid, salts and/or combinations thereof, or even any amino acids with a carboxyl group and a water soluble amino group and available in aqueous solution with a pH around 7 or lower, preferably arginine;
✔ Orthophosphoric acid: at a 0 to 40% mm proportion of the composition;
✔ Tetrasodium pyrophosphate, at a 0.5 to 40% m/m proportion of the formulation.

2. Oral composition with synergistic association of organic and inorganic components, according to claim 1, wherein phase 1 comprises:
✔ Carboxymethylcellulose, at a 0.7 to 1.0% m/m proportion of the formulation;
✔ Glycerin, at a 45 to 55% m/m proportion of the formulation;
✔ Sodium fluoride, at a 0 to 1% m/m proportion of the formulation;
✔ Sodium saccharine; at a 0.5 to 5% m/m proportion of the formulation;
✔ Sodium benzoate, at a 0.1 to 1% m/m proportion of the formulation;
✔ Xylitol, at a 0.5 to 5% m/m proportion of the formulation;
✔ Tetrasodium pyrophosphate, at a 0.5 to 40% m/m proportion of the formulation;
✔ Sorbitol, at a 40 to 70% m/m proportion of the formulation;
✔ PEG-600, at a 40 to 70% m/m proportion of the formulation;
✔ Thickener silica, at a 7 to 15% m/m proportion of the formulation;
✔ Abrasive silica, at a 7 to 15% m/m proportion of the formulation;
✔ Sodium laureth sulfate, at a 5 to 15% m/m proportion of the formulation;
✔ Triclosan, at a 0.1 to 1% m/m proportion of the formulation;
✔ Menthol, at a 1 to 5% m/m proportion of the formulation.

3. Oral composition with synergistic association of organic and inorganic components, according to claim 1, wherein phase 2 comprises:
✔ Carboxymethylcellulose, at a 0.7 to 1.0% m/m proportion of the formulation;
✔ Glycerin, at a 45 to 55% m/m proportion of the formulation;
✔ Sodium saccharine, at a 0.5 to 5% m/m proportion of the formulation;
✔ Sodium benzoate, at a 0.1 to 1% m/m proportion of the formulation;
✔ Xylitol, at a 0.1 to 5% m/m proportion of the formulation;
✔ Citric acid at a 2 to 5% m/m proportion of the formulation;
✔ Sorbitol, at a 40 to 70 m/m proportion of the formulation;
✔ Peg-600, at a 40 to 70% m/m proportion of the formulation;
✔ Thickener silica, at a 7 to 15% m/m proportion of the formulation;
✔ Mixture of microlyzed calcium carbonate at 70 to 80% and tricalcium phosphate at 20 to 30%, at a 0.5 to 5% m/m proportion of the formulation;
✔ Sodium laureth sulfate, at a 5 to 15% m/m proportion of the formulation;
✔ Triclosan, at a 0.1 to 1% m/m proportion of the formulation;
✔ Menthol, at a 0.5 to 3% m/m proportion of the formulation;
✔ pH corrector, which may be monobasic phosphate, dibasic phosphate or citric acid, as needed, at a 2 to 5% m/m proportion of the formulation.

4. Oral composition with synergistic association of organic and inorganic components, according to claim 1, wherein phase 3 comprises:
✔ Carboxymethylcellulose, at a 0.7 to 1.0% m/m proportion of the formulation;
✔ Glycerin, at a 45 to 55% m/m proportion of the formulation;
✔ Thickener silica, at a 5 to 25% m/m proportion of the formulation;
✔ Blue dye, at a 0.1 to 0.3% m/m proportion of the formulation;
✔ Phosphoric acid, at a 5 to 40% m/m proportion of the formulation.

5. Oral composition with synergistic association of organic and inorganic components, according to any one of claims 1, 2, 3 or 4, wherein its three variations are:
✔ Variation 1: professional use variation, comprising phases 1, 2 and 3, namely: 0.01 to 30% of phase 3 + 0.5 to 40% of phase 2 + 30 to 60% of phase 1;
✔ Variation 2: domestic and professional use variation, comprising phases 1 and 2, namely: 0.5 to 70% of phase 2 + 30 to 99.5% of phase 1;
✔ Variation 3: variation for domestic and daily use, comprising only phase 1, namely: 100% phase 1.

6. Oral composition with synergistic association of organic and inorganic components, according to claim 5, wherein its variations comprise pH ranging from 2 to 5.5.

7. Oral composition with synergistic association of organic and inorganic components, according to any one of claims 1 or 5, wherein the constituents may be presented in a microlyzed form, in nanometric scale.

8. Oral composition with synergistic association of organic and inorganic components, according to any one of claims 1 or 5, wherein, after application, in a completely acidic medium, said composition is electrochemically attracted by the tooth, bonding to it and causing ionization of the calcium present in its structures, thus gathering dispersed particles in the buccal medium, and condensing from calcium and other ions present in the medium, creating a hybrid layer containing silicon-enriched hydroxyapatite.

9. Oral composition with synergistic association of organic and inorganic components, according to claim 8, wherein, after application, it creates an *in situ* hybrid layer, containing silicon-enriched hydroxyapatite which bonds with dental structures.

10. Oral composition with synergistic association of organic and inorganic components, according to claim 8, wherein its action occurs in aqueous medium (mouth), with a pH between 5.5 and 7.5 *in situ.*

11. Oral composition with synergistic association of organic and inorganic components, according to any one of claims 1 or 5, wherein it is alternatively supplemented with calcium salts, using a proportion of 0.001 to 10% m/m of the composition, preferably with calcium glycerophosphate, calcium carbonate and tricalcium phosphate.

12. Oral composition with synergistic association of organic and inorganic components, according to any one of claims 1 or 5, wherein, alternatively, they are used as silicon sources: amorphous silica, bioglasses, silicates, mesoporous silica, functionalized silica, especially developed silica.

13. Oral composition with synergistic association of organic and inorganic components, according to any one of claims 1 or 5, wherein its components are presented in a single phase; phases separated by physical barriers, within a same recipient or encapsulated; or even in separated recipients.

14. Oral composition with synergistic association of organic and inorganic components, according to any one of claims 1 or 5, wherein it may be presented as: powder, liquid, cream, gel or foam dentifrice; mouthwash; drops or chewing gum.

15. Process for obtaining an oral composition with synergistic association of organic and inorganic components, according to claim 1, wherein it is performed independently for each one of the phases (phase 1, phase 2 and phase 3); therefore, after separation and weighing of components, carboxymethylcellulose at a 0.7 to 1.0% m/m proportion of the composition is added to glycerin, at a 45 to 55% m/m proportion of the composition, under slow stirring for a period of 10 to 30 minutes and speed between 45,000 and 200,000 at 25°C, obtaining the glycerin + CMC base; then the full load of deionized water is added to the reactor, at 0.1 to 7% m/m in relation to the total composition volume, activating turbine, anchor and impeller; afterwards, the remaining components of each one of the phases are added to the reactor and the process follows with minor particularities, arising out of the components used in each one of them:
✔ phase 1: salts sodium fluoride at a 0 to 1% m/m proportion of the formulation; sodium saccharine at a 0.5 to 5% m/m proportion of the formulation; sodium benzoate at a 0.1 to 1% m/m proportion of the formulation; xylitol at 0.5 to 5% m/m of formulation; tetrasodium pyrophosphate at a 0.5 to 40% m/m of the formulation, followed by homogenization for 5 to 20 minutes in the conditions previously described; followed by addition of moisteners, sorbitol, at a 40 to 70% m/m proportion of the formulation, and PEG-600 at a 40 to 70% m/m proportion of the formulation, and the previously prepared glycerin + CMC base at a 50 to 55% m/m proportion of the formulation. A vacuum application is then performed at 600 mmHg, followed by homogenization for 5 to 20 minutes in the conditions previously described; after homogenization with the vacuum turned on, the thickener silica is slowly added in a 10 to 15 minute interval at a 7 to 15% m/m proportion of the formulation; then abrasive silica is added the same way, at a 7 to 15% m/m proportion of the formulation; with the vacuum still on, homogenization is carried out for a period of 1 to 3 hours; after this period, with the vacuum still on, the sodium laureth sulfate is added at a 5 to 15% m/m proportion of the formulation; followed by homogenization for a period of 5 to 20 minutes; then triclosan is added at a 0.1 to 1% m/m proportion of the formulation, previously dissolved in menthol at a 1 to 5% m/m proportion of the formulation; followed by homogenization for a period of 5 to 20 minutes; lastly, the orthophosphoric acid is added at a 0.8 to 1.5% m/m proportion of the formulation, also with the vacuum turned on, followed by homogenization for a period of 3 to 5 hours, after which the process for obtaining phase q is finished;
✔ phase 2: sodium saccharine at a 0.5 to 5% m/m proportion of the formulation; sodium benzoate at a 0.1 to 1% m/m proportion of the formulation; xylitol at a 0.5 to 5% m/m proportion of the formulation; citric acid at a 2 to 5% m/m proportion of the formulation, followed by homogenization for 5 to 20 minutes in the conditions previously described; afterwards, moisteners and sorbitol are added at a 40 to 70 m/m proportion of the formulation, and PEG-600 at a 40 to 70% m/m proportion of the formulation, and the previously prepared glycerin + CMC base at a 50 to 55% m/m proportion of the formulation; a vacuum application is then performed at 600 mmHg, followed by homogenization for 5 to 20 minutes in the conditions described; after homogenization with the vacuum turned on, the thickener silica is slowly added in a 10 to 15 minute interval at a 7 to 15% m/m proportion of the formulation, then the calmix, at a 0.5 to 5% m/m proportion of the formulation. With the vacuum still on, homogenization is carried out for a period of 1 to 3 hours. After this period, with the vacuum still on, the sodium laureth sulfate is added at a 5 to 15% m/m proportion of the formulation; followed by homogenization for a period of 5 to 20 minutes. Then triclosan is added at a 0.1 to 1% m/m proportion of the formulation, previously dissolved in menthol at a 0.5 to 3% m/m proportion of the formulation; followed by homogenization for a period of 5 to 20 minutes. Lastly, the pH corrector is added, which may be monobasic phosphate, dibasic phosphate or citric acid, as needed, at about 2 to 5% m/m proportion of the formulation, also with the vacuum on, to adjust around a pH of 4.5 to 5.5 followed by homogenization for a period of 0.5 to 1 hour, after which the process for obtaining phase 2 is finished;
✔ phase 3: after homogenization with the vacuum turned on, the thickener silica is slowly added in a 10 to 15 minutes interval at a 5 to 25% m/m proportion of the formulation, then the blue dye, at a 0.1 to 0.3% m/m proportion of the formulation. With the vacuum still on, homogenization is carried out for a period of 1 to 2 hours; lastly, phosphoric acid is added at a 5 to 40% m/m proportion of the formulation, also with the vacuum turned on, for adjustment around a pH value of 2, followed by homogenization for a period of 0.5 to 1 hour, after which phase 3 is obtained.

16. Process for obtaining an oral composition with synergistic association of organic and inorganic components, according to claim 15, wherein the acidification of the composition occurs in pH between 2.0 and 5.5, through addition of pH correctors selected among mono- and di-sodium phosphates, phosphoric acid, citric acid and maleic acid.

17. Process for obtaining an oral composition with synergistic association of organic and inorganic components, according to claim 15, wherein it uses a very low volume of deionized water (A), between 0.1 and 7%.

18. Use of oral composition with synergistic association of organic and inorganic components, according to claims 1 or 5, wherein it is applied for full maintenance of oral health.

19. Use of oral composition with synergistic association of organic and inorganic components, according to claim 18, wherein it is applied as an anti-demineralizing agent.

20. Use of oral composition with synergistic association of organic and inorganic components, according to claim 18, wherein it is applied as a protective agent against cariogenic and microbial processes, and exposure to acids even in pH below 4.5.

21. Use of oral composition with synergistic association of organic and inorganic components, according to claim 18, wherein it is applied as a restorative agent.

22. Use of oral composition with synergistic association of organic and inorganic components, according to claim 18, wherein it is applied as an optical, chemical and mechanical whitening agent.

23. Use of oral composition with synergistic association of organic and inorganic components, according to claim 18, wherein it is applied as a protective agent against erosive processes and relief of dental sensitivity, through obliteration of dentin tubules.

24. Use of oral composition with synergistic association of organic and inorganic components, according to claim 18, wherein it is applied as protection against dental corrosion.

25. Use of oral composition with synergistic association of organic and inorganic components, according to claim 18, wherein it is day-to-day acidic attacks.
